(19) Europäisches Patentamt
European Patent Office
Office européen des brevets

(11) Veröffentlichungsnummer: **0 208 321**
**A2**

# (12) EUROPÄISCHE PATENTANMELDUNG

(21) Anmeldenummer: 86109451.4

(22) Anmeldetag: 10.07.86

(51) Int.Cl.[4]: C 07 D 249/08
C 07 D 249/10, A 01 N 43/7-07

(30) Priorität: 12.07.85 CH 3042/85
29.04.86 CH 1748/86

(43) Veröffentlichungstag der Anmeldung:
14.01.87 Patentblatt 87/3

(84) Benannte Vertragsstaaten:
AT BE CH DE FR GB IT LI LU NL SE

(71) Anmelder: F. HOFFMANN-LA ROCHE & CO.
Aktiengesellschaft

CH-4002 Basel(CH)

(72) Erfinder: Lüthy, Christoph, Dr.
Gfennstrasse 43
CH-8603 Schwerzenbach(CH)

(72) Erfinder: Zurflüh, René, Dr.
Dachslenbergstrasse 54
CH-8180 Bülach(CH)

(74) Vertreter: Lederer, Franz, Dr. et al,
Vanderwerth, Lederer & Riederer Patentanwälte
Lucile-Grahnstrasse 22
D-8000 München 80(DE)

## (54) Triazolderivate, deren Herstellung und Verwendung als Schädlingsbekämpfungsmittel.

(57) Die Erfindung betrifft neue Triazolderivate der Formel

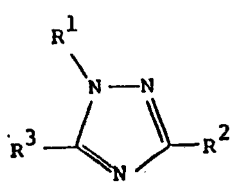

I

worin $R^1$ gegebenenfalls substituiertes Phenyl und $R^2$ substituiertes Phenyl, wie diese in der Beschreibung näher definiert sind, und $R^3$ Halogen, Methyl oder Halogenmethyl bedeuten, und ihre Säureadditionssalze, Verfahren zu ihrer Herstellung, Schädlingsbekämpfungsmittel, die diese Verbindungen als Wirkstoffe enthalten, sowie die Verwendung solcher Verbindungen bzw. Mittel zur Bekämpfung von Schädlingen.

EP 0 208 321 A2

0208321

F. HOFFMANN-LA ROCHE & CO. Aktiengesellschaft, Basel/Schweiz

RAN 6101/116

# Triazolderivate, deren Herstellung und Verwendung als Schädlingsbekämpfungsmittel

Die vorliegende Erfindung betrifft heterocyclische Verbindungen, und zwar 1,2,4-Triazole der allgemeinen Formel

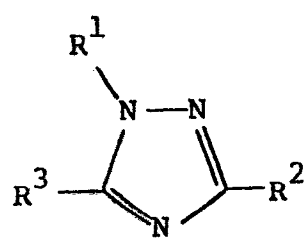

I

worin

$R^1$ gegebenenfalls mit 1 bis 3 Chloratomen, einem Bromatom, einem Jodatom, 1 bis 3 Fluoratomen, 1 oder 2 $C_{1-2}$-Alkylgruppen, 1 oder 2 Halogenmethylgruppen, einer $C_{1-2}$-Alkoxygruppe, einer $C_{1-2}$-Halogenalkoxygruppe, einer Nitrogruppe und/oder einer Cyanogruppe substituiertes Phenyl,

$R^2$ mit 1 oder 2 Chloratomen, einem Bromatom, einem Jodatom, 1 bis 3 Fluoratomen, 1 oder 2 $C_{1-2}$-Alkylgruppen, einer Halogenmethylgruppe und/oder 1 oder 2 Methoxygruppen substituiertes Phenyl, wobei sich mindestens einer der Substituenten in o-Stellung befindet, und

Pa/23.4.86

$R^3$ Halogen, Methyl oder Halogenmethyl bedeuten, sowie die Säureadditionssalze der Verbindungen der Formel I.

Die Verbindungen der Formel I und deren Säureadditionssalze sind Schädlingsbekämpfungsmittel und eignen sich insbesondere zur Bekämpfung von Insekten und Milben, z.B. Spinnmilben. Somit umfasst die Erfindung auch Schädlingsbekämpfungsmittel, welche Verbindungen der Formel I oder Säureadditionssalze davon als Wirkstoffe enthalten, ein Verfahren zur Herstellung dieser Verbindungen sowie die Verwendung dieser Verbindungen bzw. Mittel zur Bekämpfung von Schädlingen.

Der in obiger Definition der Verbindungen der Formel I verwendete Ausdruck "Halogen" umfasst Fluor, Chlor, Brom und Jod. Die Gruppen "Halogenmethyl" und "$C_{1-2}$-Halogenalkoxy" können jeweils einen oder mehrere (gleiche oder verschiedene) Halogensubstituenten aufweisen. Auch in der substituierten Phenylgruppe $R^1$ bzw. $R^2$ können die Substituenten gleich oder verschieden sein.

Als Säureadditionssalze der Verbindungen der Formel I kommen physiologisch verträgliche Salze in Frage. Hierzu gehören Salze dieser Verbindungen mit anorganischen oder organischen Säuren, vorzugsweise Halogenwasserstoffsäuren, wie Salzsäure und Bromwasserstoffsäure; Salpetersäure; Phosphorsäure; Schwefelsäure; mono- und bifunktionellen Carbonsäuren und Hydroxycarbonsäuren, wie Trifluoressigsäure und Oxalsäure; und Sulfonsäuren, wie Fluorsulfonsäure.

Eine besondere Untergruppe von Verbindungen der Formel I besteht aus denjenigen Verbindungen I, in denen $R^1$ substituiertes Phenyl bedeutet, wie dies oben näher definiert ist und wobei sich mindestens einer der Substituenten in o-Stellung befindet, und $R^2$ und $R^3$ die oben angegebenen Bedeutungen besitzen. Eine weitere besondere Untergruppe besteht aus denjenigen Verbindungen I, in denen $R^3$ Halogen, insbesondere Chlor, bedeutet und $R^1$ und $R^2$ die oben

angegebenen Bedeutungen besitzen.

$R^1$ bedeutet vorzugsweise unsubstituiertes Phenyl oder eine mono- oder disubstituierte Phenylgruppe, in der die Substituenten ein oder zwei Fluoratome, ein oder zwei Chloratome, ein Bromatom, ein Jodatom, eine oder zwei $C_{1-2}$-Alkylgruppen und/oder eine Trifluormethylgruppe sind, wobei sich vorzugsweise der oder einer der beiden Substituenten in o-Stellung befindet. Besonders bevorzugte Substituenten in o-Stellung sind Fluor, Chlor, Brom, Methyl und Trifluormethyl.

Unabhängig von der Bedeutung von $R^1$ bedeutet $R^2$ vorzugsweise eine mono- oder disubstituierte Phenylgruppe, in der die Substituenten ein oder zwei Fluoratome, ein oder zwei Chloratome, ein Bromatom und/oder ein Jodatom sind. Ganz besonders bevorzugt bedeutet $R^2$ o-Chlorphenyl, 2-Chlor-4-fluorphenyl, 2-Chlor-6-fluorphenyl oder 2,6--Difluorphenyl.

$R^3$ bedeutet unabhängig von den Bedeutungen von $R^1$ und $R^2$ vorzugsweise Fluor, Chlor, Brom oder Methyl. Ganz besonders bevorzugt bedeutet $R^3$ Chlor.

Bevorzugte einzelne Verbindungen der Formel I sind:

1,3-Bis-(o-chlorphenyl)-5-chlor-1H-1,2,4-triazol,

5-Chlor-3-(o-chlorphenyl)-1-(α,α,α-trifluor-o-tolyl)-1H-1,2,4-triazol,

5-Chlor-3-(2,6-difluorphenyl)-1-(α,α,α-trifluor-o-tolyl)-1H-1,2,4-triazol,

5-Chlor-1-(o-chlorphenyl)-3-(2-chlor-4-fluorphenyl)-1H-1,2,4-triazol,

5-Chlor-1-(o-chlorphenyl)-3-(2,6-difluorphenyl)-1H-1,2,4-triazol,

5-Chlor-3-(2-chlor-4-fluorphenyl)-1-(α,α,α-trifluor-o-tolyl)-1H-1,2,4-triazol,

5-Chlor-3-(2-chlor-6-fluorphenyl)-1-(α,α,α-trifluor-o-tolyl)-1H-1,2,4-triazol,

5-Chlor-1-(o-chlorphenyl)-3-(2-chlor-6-fluorphenyl)-1H-1,2,4-triazol,

5-Brom-3-(2,6-difluorphenyl)-1-(α,α,α-trifluor-o-tolyl)-1H-1,2,4-triazol,

3-(o-Chlorphenyl)-5-methyl-1-(α,α,α-trifluor-o-tolyl)-1H-1,2,4-triazol,

3-(2-Chlor-4-fluorphenyl)-5-methyl-1-(α,α,α-trifluor-o-tolyl)-1H-1,2,4-triazol,

3-(2,6-Difluorphenyl)-5-methyl-1-(α,α,α-trifluor-o-tolyl)-1H-1,2,4-triazol,

3-(2-Chlor-6-fluorphenyl)-5-methyl-1-(α,α,α-trifluor-o-tolyl)-1H-1,2,4-triazol,

5-Chlor-3-(o-chlorphenyl)-1-(o-tolyl)-1H-1,2,4-triazol,

5-Chlor-3-(o-chlorphenyl)-1-(3-chlor-o-tolyl)-1H-1,2,4-triazol,

5-Chlor-3-(o-chlorphenyl)-1-(5-chlor-o-tolyl)-1H-1,2,4-triazol,

5-Chlor-3-(2,6-difluorphenyl)-1-(o-tolyl)-1H-1,2,4-triazol,

5-Chlor-3-(2,6-difluorphenyl)-1-phenyl-1H-1,2,4-triazol,

5-Chlor-3-(2,6-difluorphenyl)-1-(3-chlor-o-tolyl)-1H-1,2,4-triazol,

5-Chlor-3-(2-chlor-6-fluorphenyl)-1-(3-chlor-o-tolyl)-1H-1,2,4-triazol,

1-(o-Bromphenyl)-5-chlor-3-(2,6-difluorphenyl)-1H-1,2,4-triazol,

5-Chlor-3-(o-chlorphenyl)-1-(o-fluorphenyl)-1H-1,2,4-triazol und

3-(2-Chlor-6-fluorphenyl)-5-fluor-1-(α,α,α-trifluor-o-tolyl)-1H-1,2,4-triazol.

Weitere Vertreter von Verbindungen der Formel I sind:

3-(2,6-Difluorphenyl)-5-fluor-1-(α,α,α-trifluor-o-tolyl)-1H-1,2,4-triazol,

3-(2,6-Difluorphenyl)-1-(o-tolyl)-5-trifluormethyl-1H-1,2,4-triazol,

1-(o-Aethylphenyl)-5-chlor-3-(2,6-difluorphenyl)-1H-1,2,4-triazol,

5-Chlor-1-(5-chlor-2-trifluormethylphenyl)-3-(2,6-difluorphenyl)-1H-1,2,4-triazol,

5-Chlor-1-(2-chlor-5-trifluormethylphenyl)-3-(2,6-difluorphenyl)-1H-1,2,4-triazol,

5-Chlor-1-(2-chlor-4-trifluormethylphenyl)-3-(2,6-difluorphenyl)-1H-1,2,4-triazol,

1-(o-Cyanophenyl)-3-(2,6-difluorphenyl)-5-methyl-1H-1,2,4-triazol,

5-Chlor-1-(3,5-dichlor-2,4-difluorphenyl)-3-(2,6-difluorphenyl)-1H-1,2,4-triazol,

5-Chlor-3-(2,6-difluorphenyl)-1-(o-trifluormethoxyphenyl)-1H-1,2,4-triazol,

5-Chlor-3-(2,6-difluorphenyl)-1-(α,α-difluor-o-tolyl)-1H-1,2,4-triazol,

3-(o-Bromphenyl)-5-chlor-1-(o-chlorphenyl)-1H-1,2,4-triazol,

5-Chlor-3-(2,3-dichlorphenyl)-1-(α,α,α-trifluor-o-tolyl)-1H-1,2,4-triazol,

5-Chlor-3-(2-chlor-4,6-difluorphenyl)-1-(α,α,α-trifluor-o-tolyl)-1H-1,2,4-triazol,

5-Chlor-1-(2-chlor-5-nitrophenyl)-3-(2-chlorphenyl)-1H-1,2,4-triazol,

5-Chlor-3-(o-chlorphenyl)-1-(4-nitro-2-trifluormethylphenyl)-1H-1,2,4-triazol,

1,3-Bis-(o-chlorphenyl)-5-fluor-1H-1,2,4-triazol,

5-Chlor-3-(o-methoxyphenyl)-1-(α,α,α-trifluor-o-tolyl)-1H-1,2,4-triazol,

3-(2-Chlor-4-fluorphenyl)-5-fluor-1-(α,α,α-trifluor-o-tolyl)-1H-1,2,4-triazol,

1-(o-Chlorphenyl)-3-(2-chlor-6-fluorphenyl)-5-fluor-1H-1,2,4-triazol,

5-Chlor-3-(2,6-difluorphenyl)-1-(o-fluorphenyl)-1H-1,2,4-triazol,

5-Brom-1-(o-chlorphenyl)-3-(2,6-difluorphenyl)-1H-1,2,4-triazol,

1-(o-Chlorphenyl)-3-(2,6-difluorphenyl)-5-fluor-1H-1,2,4-triazol,

5-Chlor-1-(o-chlorphenyl)-3-(2,4,6-trifluorphenyl)-1H-1,2,4-triazol,

5-Chlor-3-(2,4,6-trifluorphenyl)-1-(α,α,α-trifluor-o-tolyl)-1H-1,2,4-triazol,

5-Chlor-1-(α,α,α-chlordifluor-o-tolyl)-3-(2,6-difluorphenyl)-1H-1,2,4-triazol,

3-(o-Chlorphenyl)-5-trifluormethyl-1-(α,α,α-trifluor-o-tolyl)-1H-1,2,4-triazol,

5-Chlor-1-(o-chlorphenyl)-3-(o-tolyl)-1H-1,2,4-triazol,

5-Chlor-1-(o-chlorphenyl)-3-(α,α,α-trifluor-o-tolyl)-1H-1,2,4-triazol,

3-(o-Aethylphenyl)-5-chlor-1-(o-methoxyphenyl)-1H-1,2,4-triazol,

1-(2-Aethoxy-m-tolyl)-5-chlor-3-(o-chlorphenyl)-1H-1,2,4-triazol,

1-(o-Bromphenyl)-5-chlor-3-(o-chlorphenyl)-1H-1,2,4-triazol,

5-Chlor-3-(o-chlorphenyl)-1-(3-chlor-2,4-difluorphenyl)-1H-1,2,4-triazol,

5-Chlor-3-(o-chlorphenyl)-1-(o-trifluormethoxyphenyl)-1H-1,2,4-triazol,

5-Chlor-3-(o-chlorphenyl)-1-(p-trifluormethoxyphenyl)-1H-1,2,4-triazol,

5-Chlor-3-(o-chlorphenyl)-1-(3,5-dichlor-4-trifluormethoxyphenyl)-1H-1,2,4-triazol,

5-Chlor-3-(o-chlorphenyl)-1-(3,5-dichlor-o-tolyl)-1H-1,2,4-triazol,

5-Chlor-3-(o-chlorphenyl)-1-[3,5-di(trifluormethyl)-phenyl]-1H-1,2,4-triazol,

5-Chlor-3-(o-chlorphenyl)-1-(α,α,α-trifluor-m-tolyl)-1H-1,2,4-triazol,

5-Chlor-3-(o-chlorphenyl)-1-(3-chlor-4-trifluormethyl-phenyl)-1H-1,2,4-triazol,

5-Chlor-3-(o-chlorphenyl)-1-(2-chlor-p-tolyl)-1H-1,2,4-triazol,

5-Chlor-3-(o-chlorphenyl)-1-(3-nitro-o-tolyl)-1H-1,2,4-triazol,

5-Chlor-1-(2,3-dichlorphenyl)-3-(2,6-difluorphenyl)-1H-1,2,4-triazol,

5-Chlor-3-(2,6-difluorphenyl)-1-(p-trifluormethoxy-phenyl)-1H-1,2,4-triazol,

5-Chlor-1-[3,5-dichlor-4-(1,1,2,2-tetrafluoräthoxy)-phenyl]-3-(2,6-difluorphenyl)-1H-1,2,4-triazol,

5-Chlor-3-(2,6-difluorphenyl)-1-(4-fluor-3-trifluormethylphenyl)-1H-1,2,4-triazol,

5-Chlor-1-(4-chlor-3-trifluormethylphenyl)-3-(2,6-difluorphenyl)-1H-1,2,4-triazol,

5-Chlor-3-(2,6-difluorphenyl)-1-(p-trifluormethylphenyl)-1H-1,2,4-triazol,

5-Chlor-1-(3-chlor-4-trifluormethylphenyl)-3-(2,6-difluorphenyl)-1H-1,2,4-triazol,

5-Chlor-3-(2,6-difluorphenyl)-1-(5-fluor-o-tolyl)-1H-1,2,4-triazol,

1-(3-Chlor-o-tolyl)-3-(2,6-difluorphenyl)-5-fluor-1H-1,2,4-triazol,

1-(3-Chlor-o-tolyl)-3-(2,6-difluorphenyl)-5-methyl-1H-1,2,4-triazol,

3-(2,6-Difluorphenyl)-1-(2,3-dimethylphenyl)-5-methyl-1H-1,2,4-triazol,

5-Chlor-3-(2,6-dichlorphenyl)-1-phenyl-1H-1,2,4-triazol,

5-Chlor-3-(2,6-dichlorphenyl)-1-(o-trifluormethylphenyl)-1H-1,2,4-triazol,

5-Chlor-3-(2,6-dichlorphenyl)-1-(o-tolyl)-1H-1,2,4-triazol,

5-Chlor-3-(2-chlor-5-fluorphenyl)-1-(o-trifluormethylphenyl)-1H-1,2,4-triazol,

5-Chlor-1-(o-chlorphenyl)-3-(2-chlor-3-fluorphenyl)-1H-1,2,4-triazol,

5-Chlor-3-(2,4-dichlorphenyl)-(o-trifluormethylphenyl)-1H-1,2,4-triazol und

5-Chlor-3-(o-chlorphenyl)-1-(4-chlor-2-trifluormethylphenyl)-1H-1,2,4-triazol.

Das erfindungsgemässe Verfahren zur Herstellung der Verbindungen der Formel I und von deren Säureadditionssalzen ist dadurch gekennzeichnet, dass man

a) zwecks Herstellung derjenigen Verbindungen der Formel I, in denen $R^3$ Chlor oder Brom bedeutet, ein 1,4-Dihydro-1H-1,2,4-triazol-5-on der allgemeinen Formel

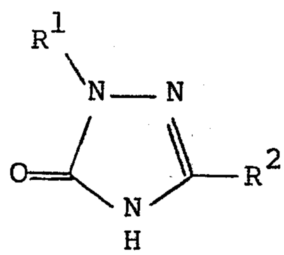

II

worin
$R^1$ und $R^2$ die oben angegebenen Bedeutungen besitzen, mit einem Chlorierungs- oder Bromierungsmittel behandelt,

b) zwecks Herstellung derjenigen Verbindungen der Formel I, in denen $R^3$ Fluor oder Jod bedeutet, ein 5-Chlor-1,2,4-triazol der allgemeinen Formel

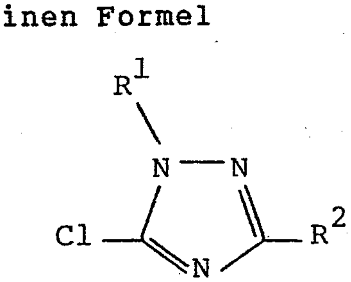

I'

worin $R^1$ und $R^2$ die oben angegebenen Bedeutungen besitzen,

einer Halogenaustauschreaktion unterwirft,

c) zwecks Herstellung derjenigen Verbindungen der Formel I, in denen $R^3$ Methyl oder Halogenmethyl bedeutet, einen

N-Acyl-benzimidsäure(thiol)ester bzw. ein N-Acyl-benzimidsäureamid der allgemeinen Formel

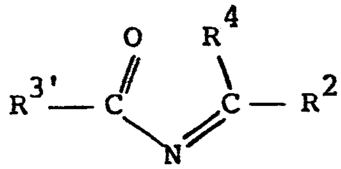 III

worin

$R^{3'}$ Methyl oder Halogenmethyl

und

$R^4$ nieder Alkoxy, nieder Alkylthio oder Di(nieder alkyl)-amino bedeuten

und

$R^2$ die oben angegebene Bedeutung besitzt,

mit einem Phenylhydrazin der allgemeinen Formel

$$R^1\text{-NH-NH}_2 \qquad \text{IV}$$

worin $R^1$ die oben angegebene Bedeutung besitzt,

oder mit einem Säureadditionssalz davon umsetzt, oder

d) zwecks Herstellung derjenigen Verbindungen der Formel I, in denen $R^3$ Methyl oder Halogenmethyl bedeutet, ein Amidrazon der allgemeinen Formel

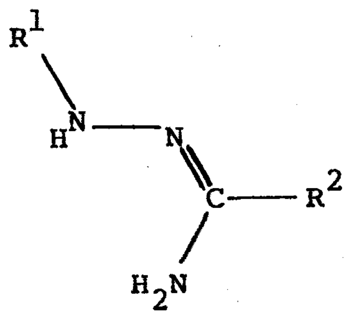 V

worin $R^1$ und $R^2$ die oben angegebenen Bedeutungen besitzen,

oder ein Säureadditionssalz davon mit einer Carbonsäure der allgemeinen Formel

$$R^{3'}\text{-COOH} \qquad \text{VI}$$

worin $R^{3'}$ die oben angegebene Bedeutung besitzt, oder mit einem reaktionsfähigen Derivat davon umsetzt,

und erwünschtenfalls eine so erhaltene Verbindung der Formel I durch Umsetzung mit einer Säure in das entsprechende Säureadditionssalz überführt.

Bei der Chlorierung bzw. Bromierung nach Verfahrensvariante a) wird zweckmässigerweise Thionylchlorid, Phosphorpentachlorid oder Phosphoroxychlorid bzw. Phosphorpentabromid oder Phosphorylbromid als Halogenierungsmittel verwendet. Gegebenenfalls wird ein Gemisch von Phosphorpentachlorid und Phosphoroxychlorid bzw. von Phosphorpentabromid und Phosphorylbromid eingesetzt, wobei ein Ueberschuss an Phosphoroxychlorid bzw. Phosphorylbromid als Verdünnungsmittel dienen kann. Die Chlorierung bzw. Bromierung kann in Gegenwart eines inerten Verdünnungsmittels, insbesondere eines aprotischen organischen Lösungsmittels, wie eines aliphatischen oder aromatischen Kohlenwasserstoffes, z.B. n-Hexan, Benzol, Toluol oder eines Xylols; eines halogenierten aliphatischen Kohlenwasserstoffes, z.B. Methylenchlorid, Chloroform oder 1,2-Dichloräthan; eines halogenierten aromatischen Kohlenwasserstoffes, z.B. Chlorbenzol, oder eines tertiären Amins, z.B. N,N-Dimethylanilin, durchgeführt werden, jedoch ist dies im Falle der Verwendung von Phosphoroxychlorid bzw. Phosphorylbromid als Halogenierungsmittel nicht notwendig. Bei Verwendung von Thionylchlorid als Halogenierungsmittel erweist es sich als zweckmässig, eine katalytische Menge Dimethylformamid zuzusetzen. Die Reaktionstemperaturen liegen im allgemeinen zwischen 0°C und der Rückflusstemperatur des Reaktionsgemisches, vorzugsweise zwischen 80°C und 120°C.

Die Halogenaustauschreaktion der Verfahrensvariante b) erfolgt vorzugsweise durch Behandlung des 5-Chlor-1,2,4-triazols der Formel I' mit einem Alkalimetall- oder Erdalkalimetall-Fluorid bzw. -Jodid, wobei die Ausdrücke

"Alkalimetall" und "Erdalkalimetall" insbesondere Natrium und Kalium bzw. Calcium und Magnesium umfassen. Die Umsetzung wird zweckmässigerweise bewerkstelligt, indem man das 5-Chlor-1,2,4-triazol in Gegenwart eines inerten Verdünnungsmittels und - im Falle der Fluoraustauschreaktion - gegebenenfalls unter Zusatz eines Phasentransferkatalysators, wie 18-Crown-6 (die Polyäthylenäther-Kronenverbindung mit einem 6 Kohlenstoffatome enthaltenden 18-Ring), mit dem Alkalimetall- oder Erdalkalimetall-Fluorid oder -Jodid behandelt. Als Verdünnungsmittel für die Fluoraustauschreaktion eignen sich insbesondere aliphatische Ketone, z.B. 2-Butanon; Aromaten, z.B. Toluol; aliphatische Nitrile, z.B. Acetonitril; und aliphatische Sulfone, z.B. Sulfolan, während als Verdünnungsmittel für die Jodaustauschreaktion insbesondere ebenfalls aliphatische Ketone, z.B. 2-Butanon, sowie auch Dimethylformamid in Frage kommen. Die Umsetzung erfolgt zweckmässigerweise bei Temperaturen zwischen ca. 20°C und der Rückflusstemperatur des Reaktionsgemisches.

Im Fall der Verfahrensvarianten c) ist unter "nieder Alkoxy", "nieder Alkylthio" bzw. "Di(nieder alkyl)-amino" ($R^4$) eine solche Gruppe zu verstehen, in welcher der bzw. jeder Alkylteil insbesondere 1 bis 6 Kohlenstoffatome enthält, vorzugsweise jedoch Methyl oder Aethyl ist. Die bevorzugten Abgangsgruppen $R^4$ sind Methoxy und Aethoxy. Zu den in Frage kommenden Säureadditionssalzen der Phenylhydrazine der Formel IV gehören Salze dieser Verbindungen mit Mineralsäuren, z.B. Salzsäure und Bromwasserstoff, sowie organischen Säuren, z.B. Oxalsäure. Vorzugsweise wird allerdings das Phenylhydrazin in Form der freien Base eingesetzt.

Die Umsetzung nach Verfahrensvariante c) erfolgt zweckmässigerweise in einem inerten Verdünnungsmittel, wie einem chlorierten aliphatischen oder aromatischen Kohlenwasserstoff, z.B. Tetrachlorkohlenstoff, 1,1,2-Trichloräthan oder 1,2-Dichlorbenzol; einem Alkohol, z.B. Aethanol oder Methylcellosolve; einem aliphatischen oder cyclischen Aether,

z.B. Diäthylenglykol-diäthyläther, Tetrahydrofuran oder Dioxan; einem aliphatischen Nitril, z.B. Acetonitril; einem aliphatischen oder aromatischen Kohlenwasserstoff, z.B. n-Heptan, Toluol oder o- oder p-Xylol; oder Dimethylformamid. Bei Verwendung eines Mineralsalzes oder eines organischen Salzes des Phenylhydrazins der Formel IV wird vorzugsweise auch noch in Gegenwart eines säurebindenden Mittels, z.B. Triäthylamin, 6-Aethyl-2-methylpyridin, 2,6--Lutidin oder Natriumacetat, umgesetzt. Die Reaktionstemperaturen können in einem grossen Bereich variiert werden, wobei im allgemeinen bei Temperaturen zwischen 20°C und der Rückflusstemperatur des Reaktionsgemisches, vorzugsweise zwischen 80°C und 120°C, gearbeitet wird.

Unter Umständen findet bei der Verfahrensvariante c) je nach Reaktionsbedingungen vorerst nur eine Hydrazinadditionsreaktion statt, und zwar unter Bildung einer Verbindung der isomeren Formeln

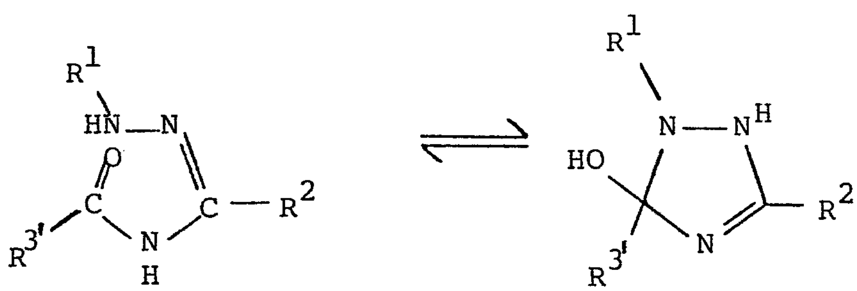

VIJa VIIb

Die Verbindung VIIa ⇌ VIIb kann nach Isolierung durch Erhitzen auf ca. 140-220°C (in der Schmelze) unter Abspaltung von Wasser in das entsprechende 5-Methyl- oder 5-Halogenmethyl-1,2,4-triazol der Formel

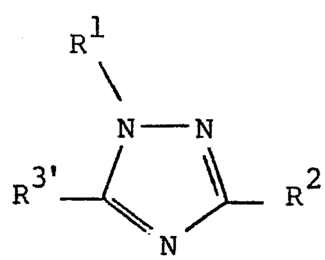

I"

übergeführt werden.

Als Alternative zur Weiterbehandlung der Verbindung VIIa⇌VIIb kann diese nach Isolierung durch Nachbehandlung mit einem wasserabspaltenden Mittel, wie Phosphoroxychlorid oder Acetanhydrid, in Gegenwart eines Verdünnungsmittels, wie eines Aromaten, z.B. Toluol, in das 1,2,4-Triazol der Formel I" übergeführt werden.

Bei der Verfahrensvariante d) wird die Carbonsäure der Formel VI bzw. ein reaktionsfähiges Derivat davon mit dem Amidrazon der Formel V zunächst amidiert, hierauf wird cyclisiert. Als reaktionsfähige Derivate der Carbonsäure der Formel VI kommen insbesondere die Säurehalogenide, wie das Säurefluorid, das Säurechlorid und das Säurebromid; das Säureanhydrid; ein Orthoester der allgemeinen Formel VIII, z.B. Orthoessigsäure-triäthylester; und ein N,N-Dimethyl--carbonsäureamid-dialkylacetal der allgemeinen Formel IX, z.B. N,N-Dimethylessigsäureamid-dimethylacetal, in Betracht:

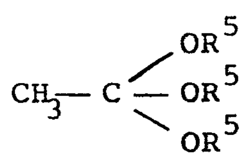

VIII

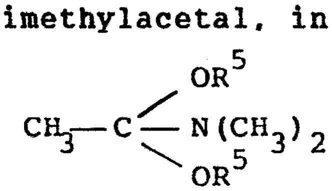

IX

worin $R^5$ $C_{1-3}$-Alkyl, insbesondere Methyl oder Aethyl, bedeutet.

Beispiele von Säureadditionssalzen der Amidrazone der Formel V sind das Hydrochlorid, das Hydrobromid und das Oxalat.

Die Umsetzung nach dieser Verfahrensvariante erfolgt zweckmässigerweise in Gegenwart eines inerten Verdünnungsmittels und - im Falle des Einsatzes einer Carbonsäure der Formel VI, eines Halogenids davon oder eines Säureadditionssalzes des Amidrazons der Formel V - zudem in Gegenwart eines säurebindenden Mittels. Beim Einsatz des Säureanhydrids der Carbonsäure ist das Vorhandensein eines säurebindenden Mittels nicht notwendig. Als Verdünnungsmittel eignen sich insbesondere die im Zusammenhang mit der Verfahrensvariante c) oben genannten sowie Pyridin. Als säurebindendes Mittel wird vorzugsweise ein tertiäres Amin, wie Triäthylamin oder Pyridin, oder ein anorganisches Salz, wie Kaliumbicarbonat, benützt. Erfolgt die Umsetzung unter Verwendung einer Carbonsäure oder von deren Anhydrid, so kann anstelle eines zugesetzten Verdünnungsmittels eine überschüssige Menge der Säure oder von deren Anhydrid selbst als Verdünnungsmittel verwendet werden. In jedem Fall liegen die Reaktionstemperaturen zweckmässigerweise zwischen 0°C und der Rückflusstemperatur des Reaktionsgemisches.

Im Falle der Verwendung des Orthoesters der Formel VIII oder des N,N-Dimethyl-carbonsäureamid-dialkylacetals der Formel IX wird eine Verbindung der Formel I hergestellt, in der $R^3$ Methyl bedeutet. Der entsprechende Reaktionsteilnehmer wird zweckmässigerweise im Ueberschuss eingesetzt. Die Umsetzung erfolgt durch Erhitzen auf ca. 100-130°C, wobei der entsprechende Alkohol $R^5OH$ freigesetzt wird. Dieser Alkohol wird zweckmässigerweise vom Reaktionsgemisch durch Abdestillieren entfernt. Zwecks Herstellung derjenigen Verbindungen der Formel I, in denen $R^3$ Methyl bedeutet, wird jedoch besonders bevorzugt das Essigsäureanhydrid mit dem Amidrazon der Formel V umgesetzt.

Zur Herstellung der Säureadditionssalze der Verbindungen der Formel I werden die Verbindungen I mit den gewünschten Säuren auf übliche Weise umgesetzt, beispielsweise durch Lösen der Verbindung der Formel I in einem geeigneten Lösungsmittel, wie Diäthyläther, Aethanol, Aethylacetat, Toluol oder Methylenchlorid, und Hinzufügen der Säure, wie Chlorwasserstoff in Form von konzentrierter oder gasförmiger Salzsäure. Der resultierende Niederschlag des Säureadditionssalzes kann anschliessend z.B. durch Filtrieren abgetrennt werden.

Die Isolierung und die Reinigung der so hergestellten Verbindungen der Formel I bzw. der Säureadditionssalze können nach an sich bekannten Methoden erfolgen. Beispielsweise kann die Verbindung der Formel I in Form ihres Säureadditionssalzes isoliert und dies mit Natronlauge zur Freisetzung des freien 1,2,4-Triazols versetzt werden, das seinerseits durch Umkristallisieren, Destillieren oder Säulenchromatographie gereinigt wird.

Die als Ausgangsmaterialien in der Verfahrensvariante a) verwendeten 1,4-Dihydro-1H-1,2,4-triazol-5-one der Formel II sind neu und können beispielsweise dadurch hergestellt werden, dass man einen Chlorameisensäure-niederalkylester der allgemeinen Formel

$$R^6\text{-COCl} \qquad X$$

worin $R^6$ nieder Alkoxy bedeutet,

mit einem Benzimidsäure(thiol)-alkylester bzw. Benzimidsäure-di(niederalkyl)amid der allgemeinen Formel

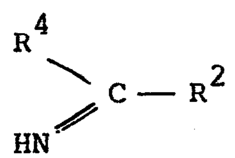 XI

worin $R^2$ und $R^4$ die oben angegebenen Bedeutungen besitzen,

umsetzt und den so hergestellten N-Alkoxycarbonyl-benzimidsäure(thiol)ester bzw. das so hergestellte N-Alkoxycarbonyl-benzimidsäureamid der allgemeinen Formel

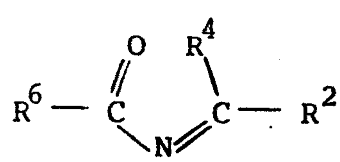 XII

worin $R^2$, $R^4$ und $R^6$ die oben angegebenen Bedeutungen besitzen,

mit einem Phenylhydrazin der oben angegebenen allgemeinen Formel IV oder mit einem Säureadditionssalz davon umsetzt.

Die Umsetzung der Verbindung der Formel X mit der Verbindung der Formel XI erfolgt zweckmässigerweise in Gegenwart einer Base, insbesondere einer sterisch gehinderten, wie 2,6-Lutidin oder sym-Collidin, sowie in Gegenwart eines inerten Verdünnungsmittels, wie eines Kohlenwasserstoffes, z.B. n-Hexan oder Petroleumbenzin, bei erhöhter Temperatur, z.B. bei der Rückflusstemperatur des Reaktionsgemisches. Dieses Herstellungsverfahren ist beispielsweise in Synthesis 1983, 483-6 exemplifiziert. Bei der in dem Ausgangsmaterial der Formel X vorhandenen niederen Alkoxygruppe $R^6$ handelt es sich insbesondere um $C_{1-6}$-Alkoxy, vorzugsweise jedoch Methoxy oder Aethoxy.

Die nachträgliche Umsetzung der in der ersten Stufe hergestellten Verbindung der Formel XII mit dem Phenylhydrazin der Formel IV oder mit einem Säureadditionssalz davon wird zweckmässigerweise unter den oben im Zusammenhang mit der Verfahrensvariante c) beschriebenen Reaktionsbedingungen durchgeführt. Auch in diesem Fall gehören zu den in Frage kommenden Säureadditionssalzen der Phenylhydrazine der Formel IV Salze mit Mineralsäuren, z.B. Salzsäure und Bromwasserstoff, sowie organischen Säuren, z.B. Oxalsäure. Vorzugsweise wird allerdings das Phenylhydrazin in Form der freien Base eingesetzt. Unter Umständen findet bei der Umsetzung je nach Reaktionsbedingungen vorerst nur eine Hydrazinadditionsreaktion statt, und zwar unter Bildung einer Verbindung der isomeren Formeln

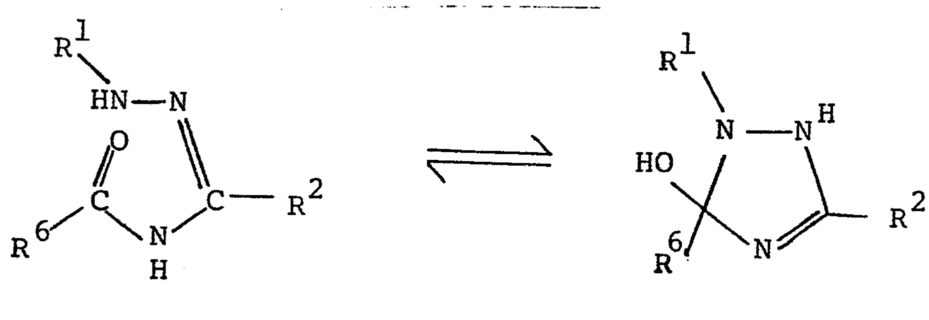

XIIIa XIIIb

Die Verbindung XIIIa⇌XIIIb kann nach Isolierung durch Erhitzen auf ca. 140-220°C (in der Schmelze) unter Abspaltung des niederen Alkanols $R^6OH$ in das entsprechende 1,4- oder 1,2-Dihydro-1H-1,2,4-triazol-5-on der Formel II bzw. II'

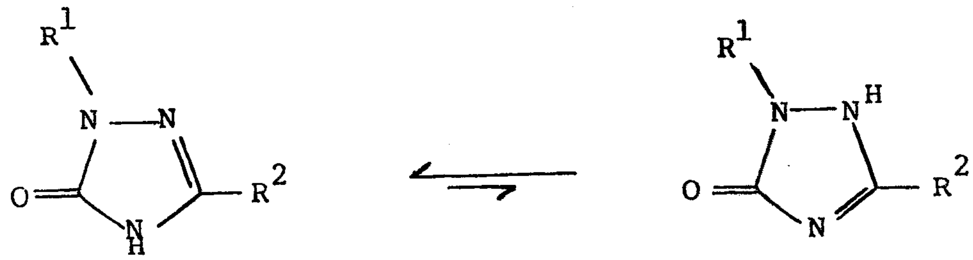

II

II'

übergeführt werden. Die Isolierung der Verbindung II⇌II' ist jedoch nicht erforderlich: Die Umsetzung des N-Alkoxycarbonyl-benzimidsäure(thiol)esters bzw. -benzimidsäureamids der Formel XII mit dem Phenylhydrazin der Formel IV erfolgt vorerst zweckmässigerweise in einem gegebenenfalls chlorierten aromatischen Kohlenwasserstoff, wie Toluol oder 1,2--Dichlorbenzol, bei Temperaturen zwischen 20°C und 80°C, und man erhitzt anschliessend auf ca. 110-160°C zur Cyclisierung und Wasserabspaltung der in situ gebildeten Verbindung XIIIa⇌XIIIb. Zweckmässigerweise wird das gebildete Alkanol $R^6H$ aus dem Reaktionsgemisch azeotrop abdestilliert.

Die 1,4-Dihydro-1H-1,2,4-triazol-5-one der Formel II können beispielsweise auch dadurch hergestellt werden, dass man ein Amidrazon der oben angegebenen allgemeinen Formel V oder ein Säureadditionssalz davon mit Phosgen oder einem Chlorameisensäure-niederalkylester, vorzugsweise einem $C_{1-3}$-Alkylester, behandelt. Beispiele von Säureadditionssalzen der Amidrazone der Formel V sind das Hydrochlorid, das Hydrobromid und das Oxalat.

Die Umsetzung wird zweckmässigerweise in Gegenwart eines inerten Verdünnungsmittels und unter Verwendung eines säurebindenden Mittels durchgeführt. Als Verdünnungsmittel eignen sich insbesondere die bei der Verfahrensvariante c) genannten sowie Pyridin. Vorzugsweise wird ein tertiäres

Amin, wie Triäthylamin oder Pyridin, als säurebindendes Mittel benützt. Auch bei der Durchführung dieser Umsetzung können die Reaktionstemperaturen in einem grossen Bereich variiert werden, wobei zweckmässigerweise bei Temperaturen zwischen 0°C und der Rückflusstemperatur des Reaktionsgemisches gearbeitet wird.

Die als Ausgangsmaterialien in der Verfahrensvariante c) verwendeten N-Acyl-benzimidsäure(thiol)ester und -amide der Formel III sind entweder bekannt oder können in an sich bekannter Weise hergestellt werden, beispielsweise durch Acylierung eines Benzimidsäure(thiol)alkylesters bzw. Benzimidsäure-di(niederalkyl)amids der oben angegebenen allgemeinen Formel XI oder eines Säureadditionssalzes davon, z.B. des Hydrochlorid- oder Tetrafluoroboratsalzes, mit einer Carbonsäure der oben angegebenen allgemeinen Formel VI oder mit einem reaktionsfähigen Derivat davon, und zwar zweckmässigerweise unter den oben im Zusammenhang mit der Verfahrensvariante d) angegebenen Reaktionsbedingungen (siehe auch z.B. Synthesis 1983, 483-6).

Die als Ausgangsmaterialien der Verfahrensvariante c) verwendeten Phenylhydrazine der Formel IV und deren Säureadditionssalze sind ebenfalls bekannt oder können nach an sich bekannten Methoden, z.B. Diazotierung (vgl. Houben-Weyl, Methoden der Organischen Chemie, Band 10/2, Seite 180ff.), hergestellt werden.

Auch die als Ausgangsmaterialien der Verfahrensvariante d) und zudem zur Herstellung der Ausgangsmaterialien II verwendeten Amidrazone der Formel V und deren Säureadditionssalze sind entweder bekannt oder können in an sich bekannter Weise hergestellt werden, beispielsweise dadurch, dass man ein Phenylhydrazin der oben angegebenen allgemeinen Formel IV oder ein Säureadditionssalz davon, z.B. das Hydrochloridsalz, mit einem Benzimidsäure-alkylester der allgemeinen Formel

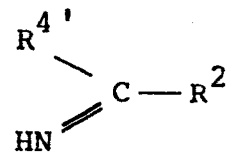

XI'

worin

$R^2$ die oben angegebene Bedeutung besitzt und

$R^{4'}$ nieder Alkoxy, vorzugsweise Methoxy oder Aethoxy, bedeutet,

oder mit einem Säureadditionssalz davon, z.B. dem Hydrochlorid- oder Tetrafluoroboratsalz, umsetzt. Die Umsetzung erfolgt zweckmässigerweise in einem inerten Verdünnungsmittel, wie einem chlorierten aliphatischen Kohlenwasserstoff, z.B. Methylenchlorid; einem Aromaten, z.B. Toluol; einem aliphatischen oder cyclischen Aether, z.B. Tetrahydrofuran oder Dioxan; einem niederen Alkanol, z.B. Aethanol; oder Pyridin, bei relativ niedrigen Temperaturen, z.B. zwischen 0°C und Raumtemperatur.

Eine weitere Methode zur Herstellung dieser Amidrazone besteht darin, dass man ein N-Phenyl-benzhydrazinoylchlorid der allgemeinen Formel

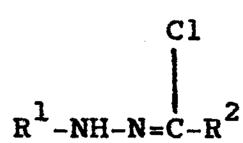

XIV

worin $R^1$ und $R^2$ die oben angegebenen Bedeutungen besitzen,

einer Aminolyse unterwirft. Dazu wird eine Lösung des N-Phenyl-benzhyrazinoylchlorids in einem inerten Lösungsmittel, wie einem Aromaten, z.B. Toluol, oder einem Aether, z.B. Diäthyläther, vorgelegt und bei ca. -40°C bis 20°C mit einer Lösung von Ammoniak in Aethanol oder Wasser bzw. mit gasförmigem Ammoniak behandelt.

Diese und weitere Methoden zur Herstellung der Amidrazone der Formel V sind u.a. in Chemical Reviews 70, 151-170 (1970) und in der dort zitierten Literatur beschrieben.

Die bei der Durchführung der obigen weiteren Methode zur Herstellung der Amidrazone V involvierten N-Phenyl-benzhydrazinoylchloride der Formel XIV sind entweder bekannt oder können nach an sich bekannten Methoden hergestellt werden, und zwar beispielsweise dadurch, indem man zunächst ein Benzoesäurechlorid der allgemeinen Formel

$$R^2COCl \qquad XV$$

worin $R^2$ die oben angegebene Bedeutung besitzt, mit einem Phenylhydrazin der oben angegebenen allgemeinen Formel IV oder einem Säureadditionssalz davon, z.B. dem Hydrochloridsalz, umsetzt und anschliessend das so erhaltene N-Phenyl-benzhydrazid der allgemeinen Formel

$$R^1\text{-NH-NH-CO-}R^2 \qquad XVI$$

mit einem Chlorierungsmittel, wie Thionylchlorid, Phosphorpentachlorid oder Phosphoroxychlorid, behandelt. Beide Reaktionsstufen können unter den dem Fachmann geläufigen Bedingungen durchgeführt werden.

Die als Ausgangsmaterialien der Verfahrensvariante b) verwendeten 1,2,4-Triazole der Formel I' sind zugleich eine Untergruppe der Verbindungen der Formel I und können gemäss Verfahrensvariante a) hergestellt werden.

Die restlichen oben erwähnten Ausgangsmaterialien, Zwischenprodukte bzw. Reagentien, also u.a. die Verbindungen der Formeln VI (und deren reaktionsfähige Derivate, z.B. die Verbindungen der Formeln VIII und IX), X, XI und XV, sind ebenfalls entweder bekannt oder können nach an sich bekannten Methoden hergestellt werden.

Die Isolierung und die Reinigung der so hergestellten Ausgangsmaterialien können nach an sich bekannten Methoden durchgeführt werden.

Die erfindungsgemässen Verbindungen, d.h. die Verbindungen der Formel I und ihre Säureadditionssalze, sind ganz allgemein als Pestizide von Wert. Sie erweisen sich als besonders wertvoll zur Bekämpfung von Insekten und Milben, insbesondere von

- Homoptera (insbesondere Blattläusen), wie z.B.
  Aphis fabae, Aphis gossypii, Aphis pomi;
  Acyrthosiphon pisum, Acyrthosiphon dirhodum;
  Brevicoryne brassicae;
  Dysaphis devecta, Dysaphis pyri, Dysaphis plantaginea;
  Macrosiphum rosae; Macrosiphum avenae;
  Myzus persicae, Myzus cerasi;
  Phorodon humuli;
  Rhopalosiphum insertum, Rhopalosiphum padi;
  Toxoptera aurantii;
  Nasonovia ribisnigri;
  Hyalopterus pruni;

- Blattflöhen (Psyllina), wie z.B. Psylla piri, Psylla pirisuga, Psylla piricola, Psylla mali;

- Weissen Fliegen, wie z.B.
  Trialeurodes vaporariorum; Aleurothrixus floccosus; Bemisia tabaci; Aleurodes proletella; Aleurocanthus woglumi; Dialeurodes citri;

- Milben, die bei dem Pflanzenschutz von Bedeutung sind, wie z.B.
  Tetranychidae (Spinnmilben), insbesondere Tetranychus urticae, Tetranychus cinnabarinus, Tetranychus

turkestani, Tetranychus McDanieli, Tetranychus kanzawai; Panonychus ulmi, Panonychus citri; Phyllocoptruta oleivora; Aculus schlechtendali; Phyllocoptes vitis; Aceria essigi, Aceria gracilis; Cecidophyopsis ribis; Eriophyes vitis; Eotetranychus sexmaculatus, Eotetranychus carpini; Hemitarsonemus latus;

- Milben, die in der Veterinärmedizin von Bedeutung sind, wie z.B. Macronyssus bursa, Macronyssus sylviarum, Macronyssus lacoti; Dermanyssus gallinae;

- Zecken, insbesondere der Familien Ixodidae und Argasidae und der Ordnungen Boophilus, Amblyomma, Hyalomma, Rhipicephalus, Ixodes, Argas und Ornithodorus.

Die erfindungsgemässen Verbindungen wirken als Kontakt- und Frassgifte. Zudem werden einige der Verbindungen von verschiedenen Pflanzen aufgenommen, so dass die zu bekämpfenden Schädlinge beim Fressen der Pflanzen vernichtet werden. Diese Verbindungen weisen also systemische Wirkung auf.

Das erfindungsgemässe Schädlingsbekämpfungsmittel ist dadurch gekennzeichnet, dass es eine wirksame Menge mindestens einer Verbindung der allgemeinen Formel I, wie oben definiert, oder eines Säureadditionssalzes einer solchen Verbindung sowie Formulierungshilfsstoffe enthält. Das Mittel enthält zweckmässigerweise zumindest einen der folgenden Formulierungshilfsstoffe:

Feste Trägerstoffe; Lösungs- bzw. Dispersionsmittel; Tenside (Netz- und Emulgiermittel); Dispergatoren (ohne

Tensidwirkung); und Stabilisatoren.

Unter Verwendung solcher und anderer Hilfsstoffe können die Verbindungen der Formel I bzw. ihre Säureadditionssalze, also die pestiziden Wirkstoffe, in die üblichen Formulierungen übergeführt werden, wie Lösungen, Suspensionen, Emulsionen, emulgierbare Konzentrate, Pasten, Schäume, Stäube, Pulver und Granulate.

Als feste Trägerstoffe kommen im wesentlichen in Frage: natürliche Mineralstoffe, wie Kaolin, Tonerden, Kieselgur, Talkum, Bentonit, Kreide, Kalkstein, Quarz, Dolomit, Attapulgit, Montmorillonit und Diatomeenerde; synthetische Mineralstoffe, wie hochdisperse Kieselsäure, Aluminiumoxid und Silikate; organische Stoffe, wie Cellulose, Stärke, Harnstoff und Kunstharze; und Düngemittel, wie Phosphate und Nitrate, wobei solche Trägerstoffe z.B. als Stäube, Pulver oder Granulate vorliegen können.

Als Lösungs- bzw. Dispersionsmittel kommen im wesentlichen in Frage: Aromaten, wie Toluol, Xylole, Benzol und Alkylnaphthaline; chlorierte Aromaten und chlorierte aliphatische Kohlenwasserstoffe, wie Chlorbenzole, Chloräthylene und Methylenchlorid; aliphatische Kohlenwasserstoffe, wie Cyclohexan und Paraffine, z.B. Erdölfraktionen; Alkohole, wie Butanol und Glykol, sowie deren Aether und Ester; Ketone, wie Aceton, Methylisobutylketon und Cyclohexanon; und stark polare Lösungsmittel, wie Dimethylformamid, N-Methylpyrrolidon und Dimethylsulfoxid, wobei solche Lösungs- bzw. Dispersionsmittel vorzugsweise Flammpunkte von mindestens 30°C und Siedepunkte von mindestens 50°C aufweisen, und Wasser. Unter den Lösungs- bzw. Dispersionsmitteln kommen auch in Frage sogenannte verflüssigte gasförmige Streckmittel oder Trägerstoffe, die solche Produkte sind, welche bei Raumtemperatur und unter Normaldruck gasförmig sind. Beispiele solcher Produkte sind insbesondere Aerosol--Treibgase, wie Halogenkohlenwasserstoffe, z.B. Dichlordifluormethan. Im Falle der Benutzung von Wasser als Lösungsmittel können z.B. auch organische Lösungsmittel als Hilfslösungsmittel verwendet werden.

Die Tenside (Netz- und Emulgiermittel) können nicht-ionische Verbindungen sein, wie Kondensationsprodukte von Fettsäuren, Fettalkoholen oder fettsubstituierten Phenolen mit Aethylenoxid; Fettsäureester und -äther von Zuckern oder mehrwertigen Alkoholen; die Produkte, die aus Zuckern oder mehrwertigen Alkoholen durch Kondensation mit Aethylenoxid erhalten werden; Blockpolymere von Aethylenoxid und Propylenoxid; oder Alkyldimethylaminoxide.

Die Tenside können auch anionische Verbindungen darstellen, wie Seifen; Fettsulfatester, z.B. Dodecylnatriumsulfat, Octadecylnatriumsulfat und Cetylnatriumsulfat; Alkylsulfonate, Arylsulfonate und fettaromatische Sulfonate, wie Alkylbenzolsulfonate, z.B. Calcium-Dodecylbenzolsulfonat, und Butylnaphthalinsulfonate; und komplexere Fettsulfonate, z.B. die Amidkondensationsprodukte von Oelsäure und N-Methyltaurin und das Natriumsulfonat von Dioctylsuccinat.

Die Tenside können schliesslich kationische Verbindungen sein, wie Alkyldimethylbenzylammoniumchloride, Dialkyldimethylammoniumchloride, Alkyltrimethylammoniumchloride und äthoxylierte quaternäre Ammoniumchloride.

Als Dispergatoren (ohne Tensidwirkung) kommen im wesentlichen in Frage: Lignin, Natrium- und Ammoniumsalze von Ligninsulfonsäure, Natriumsalze von Maleinsäureanhydrid-Diisobutylen-Copolymeren, Natrium- und Ammoniumsalze von sulfonierten Polykondensationsprodukten aus Naphthalin und Formaldehyd, und Sulfitablaugen.

Als Dispergatoren, die sich insbesondere als Verdickungs- bzw. Antiabsetzmittel eignen, können z.B. Methylcellulose, Carboxymethylcellulose, Hydroxyäthylcellulose,

Polyvinylalkohol, Alginate, Caseinate und Blutalbumin eingesetzt werden.

Beispiele von geeigneten Stabilisatoren sind säurebindende Mittel z.B. Epichlorhydrin, Phenylglycidäther und Soyaepoxide; Antioxidantien z.B. Gallussäureester und Butylhydroxytoluol; UV-Absorber z.B. substituierte Benzophenone, Diphenylacrylnitrilsäureester und Zimtsäureester; und Deaktivatoren z.B. Salze der Aethylendiaminotetraessigsäure und Polyglykole.

Die erfindungsgemässen Schädlingsbekämpfungsmittel können neben den Wirkstoffen der Formel I auch andere Wirkstoffe enthalten, z.B. anderweitige Schädlingsbekämpfungsmittel, Schädlingslockstoffe, Fungizide, Bakterizide, Herbizide, Pflanzenwachstumsregulatoren und Düngemittel. Solche Kombinationsmittel eignen sich zur Verstärkung der Aktivität bzw. zur Verbreiterung des Wirkungsspektrums. Gegebenenfalls können dadurch auch Unzulänglichkeiten von bisher bekannten zugesetzten Mitteln ausgeglichen werden.

Es wurde gefunden, dass die erfindungsgemässen Verbindungen, insbesondere die als besonders bevorzugt hervorgehobenen, mit Vorteil in Kombination mit anderen Akariziden, vor allem mit zur Bekämpfung von mobilen Stadien von Milben geeigneten, eingesetzt werden. Beispiele derartiger Akarizide sind Amitraz, Avermectin, Benzoximate, Bromopropylate, Chlorobenzilate, Cyhexatin, Dicofol, Fenbutatin oxide, Methidathion, Propargite und Aethyl-5,7-dihydro-6H-dibenz-[c,e]azepin-6-carboximidat sowie Pyrethroide mit akarizider Wirkung, wie beispielsweise Fluvalinate, Biphenthrin und Cyano-3-phenoxybenzyl-3-(2-chlor-2,3,3-trifluorprop-1-enyl)--2,2-dimethyl-cyclopropancarboxylat. Die Anwendung kann gleichzeitig als Mischung oder getrennt erfolgen. Dabei können die erfindungsgemässen Wirkstoffe den Nachteil der bekannten Akarizide mit Wirkungsschwerpunkt gegen adulte Schädlinge egalisieren, indem die nach Einsatz der bekannten Akarizide überlebenden Eier und Larven, welche sich rasch zu einer neuen schädlichen Population entwickeln können, ebenfalls abgetötet werden.

Die erfindungsgemässen Schädlingsbekämpfungsmittel enthalten im allgemeinen zwischen 0,005 und 95 Gewichtsprozent der erfindungsgemässen Verbindung(en) der Formel I als Wirkstoff(e). Sie können in einer Form vorliegen, die sich für die Lagerung und den Transport eignet. In solchen Formen, z.B. emulgierbaren Konzentraten, ist die Wirkstoffkonzentration normalerweise im höheren Bereich der obigen Konzentrationsreihe. Diese Formen können dann mit gleichen oder verschiedenen Formulierungshilfsstoffen bis zur Wirkstoffkonzentrationen verdünnt werden, die sich für den praktischen Gebrauch eignen, und solche Konzentrationen liegen normalerweise im niedrigeren Bereich der obigen Konzentrationsreihe. Emulgierbare Konzentrate enthalten im allgemeinen 5 bis 95 Gewichzsprozent, vorzugsweise 10 bis 80 Gewichtsprozent, der erfindungsgemässen Verbindung(en). Als Anwendungsformen kommen u.a. gebrauchsfertige Lösungen, Emulsionen, Suspensionen, Schäume, Pulver, Pasten, Stäubemittel und Granulate in Frage. Die Wirkstoffkonzentrationen in solchen anwendungsfertigen Zubereitungen können in grossen Bereichen variiert werden. In Spritzbrühen können z.B. Konzentrationen zwischen 0,005 und 0,5 Gewichtsprozent vorliegen. Im Ultra-Low-Volume-Verfahren können Spritzbrühen formuliert werden, in denen die Wirkstoffkonzentration vorzugsweise von 10 bis 20 Gewichsprozent beträgt, während die im Low-Volume-Verfahren und im High--Volume-Verfahren formulierten Spritzbrühen vorzugsweise eine Wirkstoffkonzentration von 0,01 bis 0,5 bzw. 0,005 bis 0,1 Gewichtsprozent aufweisen. Granulate enthalten vorzugsweise von 5 bis 50 Gewichtsprozent der erfindungsgemässen Verbindung(en) als Wirkstoff.

Die erfindungsgemässen Schädlingsbekämpfungsmittel können dadurch hergestellt werden, dass man mindestens eine

Verbindung der allgemeinen Formel I bzw. ein Säureadditionssalz einer solchen Verbindung mit Formulierungshilfsstoffen vermischt.

Die Herstellung der Mittel kann in bekannter Weise durchgeführt werden, z.B. durch Vermischen des Wirkstoffes mit festen Trägerstoffen, durch Auflösem oder Suspendieren in geeigneten Lösungs- bzw. Dispersionsmitteln, eventuell unter Verwendung von Tensiden als Netz- oder Emulgiermitteln oder von Dispergatoren, durch Verdünnen bereits vorbereiteter emulgierbarer Konzentrate mit Lösungs- bzw. Dispersionsmitteln, usw.

Im Falle von pulverförmigen Mitteln kann der Wirkstoff mit einem festen Trägerstoff vermischt werden, z.B. durch Zusammenmahlen; oder man kann den festen Trägerstoff mit einer Lösung oder Suspension des Wirkstoffes imprägnieren und dann das Lösungs- oder Suspensionsmittel durch Abdunsten, Erhitzen oder durch Absaugen unter vermindertem Druck entfernen. Durch Zusatz von Tensiden bzw. Dispergatoren kann man solche pulverförmige Mittel mit Wasser leicht benetzbar machen, so dass sie in wässrige Suspensionen, die sich z.B. als Spritzmittel eignen, übergeführt werden können.

Die Verbindungen der Formel I bzw. deren Säureadditionssalze können auch mit einem Tensid und einem festen Trägerstoff zur Bildung eines netzbaren Pulvers vermischt werden, welches in Wasser dispergierbar ist, oder sie können mit einem festen vorgranulierten Trägerstoff zur Bildung eines granulatförmigen Produktes vermischt werden.

Wenn gewünscht, kann die Verbindung der Formel I oder ein Säureadditionssalz davon in einem mit Wasser nicht mischbaren Lösungsmittel, wie beispielsweise einem alicyclischen Keton, gelöst werden, das zweckmässigerweise gelöste Emulgiermittel enthält, so dass die Lösung bei Zugabe zu Wasser selbstemulgierend wirkt. Andernfalls kann der Wirkstoff mit einem Emulgiermittel vermischt und das Gemisch dann mit Wasser auf die gewünschte Konzentration verdünnt werden. Zudem kann der Wirkstoff in einem Lösungsmittel gelöst und danach die Lösung mit einem Emulgiermittel gemischt werden. Ein solches Gemisch kann ebenfalls mit Wasser auf die gewünschte Konzentration verdünnt werden. Auf diese Weise erhält man emulgierbare Konzentrate bzw. gebrauchsfertige Emulsionen.

Das erfindungsgemässe Verfahren zur Bekämpfung von Schädlingen ist dadurch gekennzeichnet, dass man das zu schützende Gut oder die Schädlinge selbst mit einer wirksamen Menge einer erfindungsgemässen Verbindung bzw. eines erfindungsgemässen Schädlingsbekämpfungsmittels behandelt. Dieses Anwendungsverfahren kann durch Boden- oder Blattapplikation, bzw. durch Applikation auf die zu schützenden Tiere, Vorräte oder Materialien, je nach Art der zu bekämpfenden Schädlinge, durchgeführt werden. Die Bekämpfung wird beispielsweise durch Kontakt oder durch Einnahme mit der Nahrung erzielt.

Die Anwendung kann in konventioneller Weise geschehen, z.B. durch Verspritzen, Versprühen, Vernebeln, Verstäuben, Verstreuen, Eindrillen, Verräuchern, Giessen, Beizen oder Inkrustieren. Pulverförmige Präparate können z.B. als Stäubemittel mit Hilfe der üblichen Verstäubegeräte auf die Schädlinge bzw. auf das zu schützende Gut, z.B. Pflanzen oder Tiere, aufgebracht werden. Wässrige Suspensionen sind z.B. als Spritzmittel anwendbar.

Bei der Anwendung im Pflanzenschutz genügt in der Regel eine Dosierung von ca. 120-500 g Wirkstoff [Verbindungen(en) der Formel I]/ha, z.B. wie dies bei der Applikation von 2000 l einer Spritzbrühe, die 0,006-0,025 Gewichtsprozent Wirkstoff enthält, auf 1 ha bepflanzten Erdbodens der Fall ist.

Die nachfolgenden Beispiele dienen zur näheren Erläuterung der Erfindung.

I. Herstellung der Wirkstoffe der Formel I:

## Beispiel 1

Ein Gemisch von 9,8 g (32 mMol) 1,3-Bis-(o-chlorphenyl)-1,4-dihydro-1H-1,2,4-triazol-5-on und 7,3 g (35 mMol) Phosphorpentachlorid in 20 ml Phosphoroxychlorid wird während 24 Stunden auf 110°C erhitzt. Danach fügt man noch 2,97 g (14 mMol) Phosphorpentachlorid zu und erhitzt das Gemisch weitere 22 Stunden auf Rückflusstemperatur. Man tropft das abgekühlte Reaktionsgemisch vorsichtig unter Temperaturkontrolle auf Wasser bei 30-35°C, neutralisiert das wässrige Gemisch mit 30%iger Natronlauge und extrahiert es zweimal mit 150 ml Diäthyläther. Die vereinigten Extrakte werden einmal mit gesättiger Natriumchloridlösung gewaschen, über wasserfreiem Magnesiumsulfat getrocknet und unter vermindertem Druck eingedampft. Durch Eluieren des Rückstandes mit n-Hexan/Aethylacetat (17:3) an Kieselgel (Ø 40-63 µm) und nachfolgendes Umkristallisieren aus Aethylacetat/n-Hexan erhält man reines Produkt, und zwar das 1,3-Bis-(o-chlorphenyl)-5-chlor-1H-1,2,4-triazol, Smp. 95,5°C; Massenspektrum: 323(66), 262(24), 125(100).

Das 1,3-Bis-(o-chlorphenyl)-5-chlor-1H-1,2,4-triazol kann auch wie folgt hergestellt werden:

Eine Suspension von 13,7 g (45 mMol) 1,3-Bis-(o-chlorphenyl)-1,4-dihydro-1H-1,2,4-triazol-5-on in 40 ml (0,44 Mol) Phosphoroxychlorid wird 24 Stunden auf Rückflusstemperatur erhitzt. Dann tropft man das klar gewordene Reaktionsgemisch vorsichtig mit Temperaturkontrolle auf Wasser bei 30-35°C. Anschliessend wird das Gemisch mit 28%iger Natronlauge neutralisiert und zweimal mit je 150 ml Diäthyläther extrahiert. Man wäscht die vereinigten Extrakte mit gesättigter Natriumchloridlösung, trocknet sie über wasserfreiem Magnesiumsulfat und dampft sie unter vermindertem Druck ein. Nach Destillieren des Rückstandes im Kugelrohr bei 175°C/0,1 mmHg (13,33 Pa) erhält man reines 1,3-Bis-(o-chlorphenyl)-5-chlor-1H-1,2,4-triazol, Smp. 95,5°C; Massenspektrum: 323(35), 262(14), 125(100).

Beispiele 2-36

Analog den in Beispiel 1 beschriebenen Verfahren werden die entsprechenden 1,4-Dihydro-1H-1,2,4-triazol-5-one der Formel II chloriert bzw. bromiert, um die in der nachstehenden Tabelle 1 aufgeführten Verbindungen der Formel I herzustellen.

Tabelle 1

| Beispiel | $R^1$ | $R^2$ | $R^3$ | Physikalische Daten |
|---|---|---|---|---|
| 2 | α,α,α-Trifluor-o-tolyl | o-Chlorphenyl | Chlor | Sdp. 160°C/0,05 mmHg (6,67 Pa);Massenspektrum: 357(80), 296(37), 159(100) |
| 3 | " | 2,6-Difluorphenyl | " | Smp. 100-100,5°C Massenspektrum: 359(69), 298(57), 159(100) |
| 4 | o-Chlorphenyl | 2-Chlor-4-fluorphenyl | " | Smp. 103,5°C Massenspektrum: 341(16), 280(5), 125(100) |
| 5 | " | 2,6-Difluorphenyl | " | Smp. 94°C Massenspektrum: 325(10), 264(5), 125(100) |
| 6 | α,α,α-Trifluor-o-tolyl | 2-Chlor-4-fluorphenyl | " | Smp. 87°C Massenspektrum: 375(45), 314(17), 159(100) |
| 7 | " | 2-Chlor-6-fluorphenyl | " | Smp. 76°C Massenspektrum: 375(48), 314(41), 159(100) |
| 8 | o-Chlorphenyl | " | " | Smp. 85°C Massenspektrum: 341(22), 280(11), 125(100) |
| 9 | 3,4-Dichlorphenyl | 2,6-Difluorphenyl | " | Smp. 143,5°C Massenspektrum: 359(39), 298(15), 159(100) |

Tabelle 1 (Fortsetzung)

| Beispiel | $R^1$ | $R^2$ | $R^3$ | Physikalische Daten |
|---|---|---|---|---|
| 10 | o-Tolyl | o-Chlorphenyl | Chlor | Sdp. 165°C/0,06 mmHg (8,00 Pa) Massenspektrum: 303(17), 268(100), 131(48) |
| 11 | α,α,α-Trifluor-o-tolyl | o-Jodphenyl | " | Sdp. 210°C/0,05 mmHg (6,67 Pa) Massenspektrum: 449(100), 388(18), 159(40) |
| 12 | 2-Chlor-6-methylphenyl | o-Chlorphenyl | " | Smp. 76°C Massenspektrum: 337(30), 302(100) |
| 13 | 4-Chlor-o-tolyl | " | " | Smp. 70,5°C Massenspektrum: 337(24), 302(99) |
| 14 | 3-Chlor-o-tolyl | " | " | Sdp. 175°C/0,06 mmHg (8,00 Pa) Massenspektrum: 337(26), 302(100) |
| 15 | 5-Chlor-o-tolyl | " | " | Smp. 89°C Massenspektrum: 337(29), 302(100) |
| 16 | 2-Chlor-5-trifluor-methylphenyl | " | " | Sdp. 175°C/0,08 mmHg (10,67 Pa) Massenspektrum: 391(53), 330(20), 193(100) |

<u>Tabelle 1</u> (Fortsetzung)

| Beispiel | $R^1$ | $R^2$ | $R^3$ | Physikalische Daten |
|---|---|---|---|---|
| 17 | 2-Chlor-6-fluorphenyl | o-Chlorphenyl | Chlor | Smp. 88-89°C<br>Massenspektrum: 341(42), 280(10), 143(100) |
| 18 | o-Tolyl | 2,6-Difluorphenyl | " | Sdp. 180°C/0,055 mmHg (7,34 Pa)<br>Massenspektrum: 305(18), 270(100), 131(47) |
| 19 | Phenyl | " | " | Smp. 59°C<br>Massenspektrum: 291(35), 230(14), 91(100) |
| 20 | 3,5-Dichlorphenyl | " | " | Smp. 121°C<br>Massenspektrum: 359(72), 298(46), 159(100) |
| 21 | 2,4-Dimethylphenyl | " | " | Sdp. 150°C/0,05 mmHg (6,67 Pa); $^1$H-NMR ($CDCl_3$,60 MHz): 2,19 (s,$C\underline{H}_3$), 2,41 (s,$C\underline{H}_3$)<br>Massenspektrum: 319(23), 284(100), 145(41) |
| 22 | Phenyl | o-Chlorphenyl | " | Smp. 86°C<br>Massenspektrum: 289(39), 228(13), 91(100) |
| 23 | α,α,α-Trifluor-m-tolyl | 2,6-Dimethoxyphenyl | " | Smp. 170-172°C<br>$^1$H-NMR ($CDCl_3$, 60 MHz): 3,81 (s,2x$OC\underline{H}_3$); Massenspektrum: 383(70) |

Tabelle 1 (Fortsetzung)

| Beispiel | $R^1$ | $R^2$ | $R^3$ | Physikalische Daten |
|---|---|---|---|---|
| 24 | α,α,α-Trifluor-m-tolyl | 2,6-Difluor-phenyl | Chlor | Smp. 77-79°C<br>Massenspektrum: 359(46), 298(34), 159(100) |
| 25 | 3-Chlor-o-tolyl | " | " | Smp. 100-102°C<br>$^1H$-NMR ($CDCl_3$, 60 MHz): 2,22 (s,$C\underline{H}_3$)<br>Massenspektrum: 339(25), 304(100) |
| 26 | " | 2-Chlor-6-fluorphenyl | " | Smp. 74-76°C<br>$^1H$-NMR ($CDCl_3$, 60 MHz): 2,24 (s,$C\underline{H}_3$)<br>Massenspektrum: 357(21), 320(100) |
| 27 | 2,3-Dimethyl-phenyl | o-Chlorphenyl | " | Smp. 69°C<br>$^1H$-NMR ($CDCl_3$, 60 MHz): 2,09 (s,$C\underline{H}_3$), 2,39 (s, $C\underline{H}_3$) |
| 28 | " | 2,6-Difluor-phenyl | " | Smp. 105°C<br>$^1H$-NMR ($CDCl_3$, 60 MHz): 2,04 (s,$C\underline{H}_3$), 2,37 (s,$C\underline{H}_3$); Massen-spektrum: 319(21), 284(100) |
| 29 | m-Fluorphenyl | o-Chlorphenyl | " | Smp. 73°C<br>Massenspektrum: 307(66), 246(50), 109(100) |

Tabelle 1 (Fortsetzung)

| Beispiel | $R^1$ | $R^2$ | $R^3$ | Physikalische Daten |
|---|---|---|---|---|
| 30 | o-Nitrophenyl | o-Chlorphenyl | Chlor | Smp. 102°C<br>Massenspektrum: 335(17) |
| 31 | o-Bromphenyl | 2,6-Difluor-phenyl | " | Smp. 99-100°C<br>Massenspektrum: 369/371 (52), 308/310(9), 90(100) |
| 32 | o-Methoxy-phenyl | o-Chlorphenyl | " | Oel<br>$^1$H-NMR ($CDCl_3$, 60 MHz): 3,87 (s, $OC\underline{H}_3$); Massenspektrum: 319(21), 284(100) |
| 33 | o-Fluorphenyl | " | " | Smp. 70-72°C<br>Massenspektrum: 307(32), 246(12), 109(100) |
| 34 | 3-Chlor-2-cyanophenyl | " | " | Smp. 174-175°C<br>Massenspektrum: 348(97), 287(12), 150(100) |
| 35 | o-Cyanophenyl | 2,6-Difluor-phenyl | " | Smp. 155°C<br>IR: 2238 $cm^{-1}$; Massen-spektrum: 316(21), 297(14), 255(25), 116(100) |
| 36 | α,α,α-Trifluor-o-tolyl | " | Brom | Smp. 73-74°C<br>Massenspektrum: 403/405 (46), 298(67), 159(100) |

Beispiel 37

Ein Gemisch von 2,61 g (6,9 mMol) 5-Chlor-3-(2-chlor-6--fluorphenyl)-1 -(α,α,α-trifluor-o-tolyl)-1H-1,2,4--triazol, 0,84 g (14,5 mMol) trockenem Kaliumfluorid und 0,23 g (0,9 mMol) 18-Crown-6 in 15 ml trockenem Sulfolan wird während 65 Stunden auf 140°C erhitzt. Das Reaktionsgemisch wird anschliessend gekühlt und an Kieselgel (Ø 40--63 µm, Säule: Ø 7 cm, Länge 20 cm) mit Aethylacetat/n-Hexan (7:13) eluiert. Man erhält das 3-(2-Chlor-6-fluorphenyl)-5-fluor -1-(α,α,α-trifluor-o-tolyl)-1H-1,2,4--triazol als weisse Kristalle, Smp. 68-70°C; Massenspektrum: 359(100).

Beispiel 38

Ein Gemisch von 11,3 g (50 mMol) N-Acetyl-o-chlorbenzimidsäure-äthylester und 8,8 g (50 mMol) o-Trifluormethylphenylhydrazin in 60 ml Tetrachlorkohlenstoff wird während 3 Stunden auf Rückflusstemperatur erhitzt. Das Reaktionsgemisch wird dann mit 240 ml Methylenchlorid verdünnt und das Ganze mit 100 ml Wasser gewaschen, und die organische Phase wird anschliessend über wasserfreiem Magnesiumsulfat getrocknet und unter vermindertem Druck eingedampft.

Man erhält dabei 15,6 g Rückstand, welcher nunmehr in 80 ml Toluol aufgenommen und zusammen mit 4,6 ml (50 mMol) Phosphoroxychlorid 1 Stunde auf Rückflusstemperatur erhitzt wird. Man nimmt anschliessend das Gemisch in 200 ml Diäthyläther auf und wäscht die Lösung je einmal mit 5%iger Natriumbicarbonatlösung und gesättigter Natriumchloridlösung, trocknet sie über wasserfreiem Magnesiumsulfat und dampft sie unter vermindertem Druck ein. Der Rückstand wird durch Chromatographie an Kieselgel (Ø 40-60 µm) mit n-Hexan/Aceton (3:1) gereinigt und anschliessend einer

Kugelrohrdestillation unterworfen, wobei die Fraktion mit Sdp. 180°C/0,1 mmHg (13,33 Pa) gesammelt wird. Auf diese Weise erhält man als gelborangefarbenes Oel das 3-(o-Chlorphenyl)-5-methyl-1-(α,α,α-trifluor-o-tolyl) -1H-1,2,4-triazol, $^{1}H$-NMR ($CDCl_3$, 60 MHz): 2,38 (s,C$\underline{H}_3$); Massenspektrum: 337(46), 296(53), 159(100).

## Beispiel 39

Ein Gemisch von 6,1 g (22 mMol) N-Acetyl-2-chlor-4-fluorbenzimidsäure-äthylester und 6,7 g (25 mMol) o-Trifluormethylphenylhydrazin-hydrochlorid in 100 ml Toluol wird mit 3,9 ml (28 mMol) Triäthylamin versetzt, und das Ganze wird 18 Stunden auf Rückflusstemperatur erhitzt. Danach wäscht man das Reaktionsgemisch einmal mit Wasser und extrahiert es zweimal mit 50 ml konzentrierter Salzsäure. Die sauren wässrigen Phasen werden mit eiskalter Natronlauge neutralisiert und zweimal mit Diäthyläther extrahiert, und die vereinigten Extrakte über wasserfreiem Magnesiumsulfat getrocknet und unter vermindertem Druck eingedampft. Der Rückstand wird einer Kugelrohrdestillation unterworfen, wobei die Fraktion mit Sdp. 200°C/0,07 mmHg (9,33 Pa) gesammelt wird, und schliesslich aus Diisopropyläther/n-Hexan kristallisiert. Man erhält auf diese Weise das 3-(2-Chlor-4-fluorphenyl)-5-methyl-1-(α,α,α-trifluor-o-tolyl)-1H-1,2,4-triazol, Smp. 116°C; $^{1}H$-NMR ($CDCl_3$, 60 MHz): 2,37 (s,C$\underline{H}_3$); Massenspektrum: 355(48), 314(43), 159(100).

## Beispiele 40-54

Analog dem in Beispiel 38 oder 39 beschriebenen Verfahren werden die entsprechenden Ausgangsmaterialien der Formeln III und IV (freies Hydrazin bzw. Säureadditionssalz davon) miteinander umgesetzt, um die in der nachstehenden Tabelle 2 aufgeführten Verbindungen der Formel I herzustellen.

Tabelle 2

| Beispiel | $R^1$ | $R^2$ | $R^3$ | Physikalische Daten |
|---|---|---|---|---|
| 40 | α,α,α-Trifluor-o-tolyl | o-Fluorphenyl | Methyl | Sdp. 200°C/0,05 mmHg (6,67 Pa) $^1$H-NMR($CDCl_3$,60 MHz): 2,37 (s,C$\underline{H}_3$) |
| 41 | " | 2,4-Dichlorphenyl | " | Smp. 95-96°C $^1$H-NMR($CDCl_3$,60 MHz): 2,42 (s,C$\underline{H}_3$) Massenspektrum: 371(44), 330(44), 159(100) |
| 42 | " | o-Bromphenyl | " | Smp. 67-68,5°C $^1$H-NMR($CDCl_3$,60 MHz): 2,38 (s,C$\underline{H}_3$) Massenspektrum: 381/383(28), 340/342(22), 159(100) |
| 43 | " | o-Tolyl | " | Oel $^1$H-NMR($CDCl_3$,60 MHz): 2,37 (s,C$\underline{H}_3$), 2,63 (s,C$\underline{H}_3$) Massenspektrum: 317(100), 159(60) |
| 44 | " | o-Methoxyphenyl | " | Sdp. 200°C/0,05 mmHg (6,67 Pa) $^1$H-NMR($CDCl_3$,60 MHz): 2,35 (s, C$\underline{H}_3$), 3,91 (s,OC$\underline{H}_3$) Massenspektrum: 333(41), 304(48), 262(28), 159(78) |

<u>Tabelle 2</u> (Fortsetzung)

| Beispiel | $R^1$ | $R^2$ | $R^3$ | Physikalische Daten |
|---|---|---|---|---|
| 45 | 4-Brom-2-trifluormethylphenyl | o-Chlorphenyl | Methyl | Smp. 82-84°C<br>$^1H$-NMR($CDCl_3$,60 MHz):<br>2,39 (s,$C\underline{H}_3$)<br>Massenspektrum:<br>415/417(54),<br>374/376(50), 237/239(100) |
| 46 | o-Fluorphenyl | " | " | Smp. 67°C<br>$^1H$-NMR($CDCl_3$,60 MHz):<br>2,50 (d,J=2Hz,$C\underline{H}_3$)<br>Massenspektrum: 287(28),<br>246(25), 109(100) |
| 47 | o-Bromphenyl | 2,6-Difluorphenyl | " | Smp. 125°C<br>$^1H$-NMR($CDCl_3$,60 MHz):<br>2,45 (s,$C\underline{H}_3$)<br>Massenspektrum:<br>349/351(37),<br>308/310(27), 169/171(84) |
| 48 | o-Chlorphenyl | α,α,α-Trifluor-o-tolyl | " | Sdp. 175°C/0,03 mmHg<br>(4,00 Pa)<br>$^1H$-NMR($CDCl_3$,60 MHz):<br>2,42 (s,$C\underline{H}_3$)<br>Massenspektrum: 337(29),<br>296(21), 125(100) |
| 49 | " | o-Chlorphenyl | Chlormethyl | Smp. 98,5°C; $^1H$-NMR<br>($CDCl_3$,60 MHz): 4,63<br>(s,$C\underline{H}_2Cl$); Massenspektrum: 339(31), 337(29),<br>262(30), 125(100) |

Tabelle 2 (Fortsetzung)

| Beispiel | $R^1$ | $R^2$ | $R^3$ | Physikalische Daten |
|---|---|---|---|---|
| 50 | o-Tolyl | 2,6-Difluor-phenyl | Methyl | Smp. 97-98°C $^1$H-NMR ($CDCl_3$, 60 MHz): 2,17 (s, $C\underline{H}_3$), 2,44 (s,$C\underline{H}_3$) Massenspektrum: 285(89), 270(34), 244(6), 105(100) |
| 51 | α,α,α-Trifluor-o-tolyl | o-Jodphenyl | " | Smp. 71-73°C $^1$H-NMR ($CDCl_3$, 60 MHz): 2,39 (s,$C\underline{H}_3$) Massenspektrum: 429(86), 388(42), 159(100) |
| 52 | α,α,α-Trifluor-m-tolyl | 2,6-Difluor-phenyl | " | $^1$H-NMR ($CDCl_3$, 60 MHz): 2,68 (s,$C\underline{H}_3$) Massenspektrum: 339(49), 298(50), 159(100) |
| 53 | Phenyl | " | " | Sdp. 210°C/0,15 mmHg (20,00 Pa) $^1$H-NMR ($CDCl_3$, 60 MHz): 2,64 (s,$C\underline{H}_3$) Massenspektrum: 271(40), 230(31), 91(100) |
| 54 | o-Chlorphenyl | o-Chlorphenyl | " | Smp. 74-76°C $^1$H-NMR ($CDCl_3$, 60 MHz): 2,45 (s,$C\underline{H}_3$) Massenspektrum: 337(29), 296(21), 125(100) |

Beispiel 55

Ein Gemisch von 5,1 g (23 mMol) N-Acetyl-o-chlorbenzimidsäure-äthylester und 3,8 g (25 mMol) o-Nitrophenylhydrazin in 200 ml Tetrahydrofuran wird 3 Tage auf Rückflusstemperatur erhitzt. Dann dampft man das Lösungsmittel ab, nimmt den Rückstand in Methylenchlorid auf und wäscht die Lösung mit 5%iger Natriumbicarbonatlösung. Die organische Phase wird über wasserfreiem Magnesiumsulfat getrocknet und unter vermindertem Druck eingedampft. Durch Eluieren des Rückstands mit n-Hexan/Aceton (3:1) an Kieselgel (Ø 40-63 µm) und nachfolgendes Kristallisieren erhält man reineres Zwischenprodukt, und zwar das N-Acetyl-N'-(o-nitrophenylhydrazino)-o-chlorbenzimidsäureamid als orangefarbenes Kristallisat, Smp. 196-198°C.

2,85 g (8,6 mMol) des obigen Zwischenproduktes und 1,1 ml (12 mMol) Phosphoroxychlorid werden 2 Stunden in 15 ml Toluol auf Rückflusstemperatur erhitzt. Man nimmt anschliessend das Gemisch in 60 ml Aethylacetat auf, extrahiert die Lösung zweimal mit 30 ml konzentrierter Salzsäure, stellt die sauren wässrigen Phasen auf pH 2-3 mit eiskalter Natronlauge und extrahiert sie zweimal mit 60 ml Aethylacetat. Die vereinigten Extrakte werden je einmal mit eiskalter verdünnter Natronlauge und gesättigter Natriumchloridlösung gewaschen, über wasserfreiem Natriumsulfat getrocknet und unter vermindertem Druck eingedampft, und der Rückstand wird dann zweimal in heisses Aceton aufgenommen und die vereinigten Lösungen mit Aktivkohle behandelt und zum Ausfallen des Produktes mit n-Hexan versetzt. Auf diese Weise erhält man das 3-(o-Chlorphenyl)-5-methyl-1-(o-nitrophenyl)-1H-1,2,4-triazol als orangefarbene Kristalle, Smp. 108-109°C; $^{1}$H-NMR ($CDCl_3$, 60 MHz): 2,49 (s,$C\underline{H}_3$); Massenspektrum: 314(36), 139(100).

**Beispiel 56**

Zu einem Gemisch von 3,4 g (11 mMol) $N^1$-(α,α,α-Trifluor-o-tolyl)-2,6-difluorbenzamidrazon und 0,9 ml (12,6 mMol) Acetylchlorid in 60 ml Toluol bei 60°C werden 1,7 ml (12,3 mMol) Triäthylamin zugetropft. Man erhitzt dann das Gemisch langsam auf Rückflusstemperatur und hält es 18 Stunden bei dieser Temperatur. Anschliessend wird das Reaktionsgemisch vorerst einmal mit Wasser gewaschen und dann viermal mit je 10 ml konzentrierter Salzsäure extrahiert. Die vereinigten, sauren wässrigen Extrakte werden mit eiskalter Natronlauge neutralisiert und zweimal mit je 50 ml Diäthyläther extrahiert, und man trocknet die vereinigten organischen Phasen über wasserfreiem Magnesiumsulfat und dampft sie unter vermindertem Druck ein. Der Rückstand wird durch Chromatographie an Kieselgel (Ø 40-63 µm) mit n-Hexan/Aethylacetat (3:2) gereinigt und anschliessend aus Diisopropyläther/n-Hexan umkristallisiert. Auf diese Weise erhält man als weisses Kristallisat das 3-(2,6-Difluorphenyl)-5-methyl-1-(α,α,α-trifluor-o-tolyl)-1H-1,2,4-triazol, Smp. 80-82°C; $^1$H-NMR ($CDCl_3$, 60 MHz): 2,42 (s,C$\underline{H}_3$); Massenspektrum: 339(49), 298(56), 159(100).

Das 3-(2,6-Difluorphenyl)-5-methyl-1-(α,α,α-trifluor-o-tolyl)-1H-1,2,4-triazol kann auch wie folgt hergestellt werden:

Eine Lösung von 72,5 g (0,23 Mol) $N^1$-(α,α,α-Trifluor-o-tolyl)-2,6-difluorbenzamidrazon in 143 g (1,4 Mol) Acetanhydrid wird während 3 Stunden auf Rückflusstemperatur erhitzt und das Reaktionsgemisch anschliessend unter vermindertem Druck eingedampft. Man nimmt den Rückstand in ca. 300 ml Methylenchlorid auf, wäscht die Lösung je einmal mit verdünnter Natronlauge und halbgesättigter Natriumchloridlösung, trocknet sie über wasserfreiem Magnesiumsulfat und dampft sie unter vermindertem Druck ein. Schliesslich kristallisiert man das Rohprodukt einmal aus Methylenchlorid/n-Hexan um. Auf diese Weise erhält man das 3-(2,6-Difluorphenyl)-5-methyl-1-(α,α,α-trifluor -o-tolyl)-1H-1,2,4-triazol, Smp. 82-82,5°C.

Eine ätherische Lösung des obigen Produktes wird mit trockenem Chlorwasserstoff gesättigt. Man filtriert die ausgefallenen Kristalle ab und wäscht sie gut mit Diäthyläther. Auf diese Weise erhält man das 3-(2,6-Difluorphenyl)-5-methyl -1-(α,α,α-trifluor-o-tolyl)-1H-1,2,4-triazol-hydrochlorid, Smp. 160-165°C.

Beispiel 57

Eine Lösung von 41,7 g (0,13 Mol) $^{1}$N-(α,α,α-Trifluor-o-tolyl)-2-chlor -6-fluorbenzamidrazon in 77 g (0,75 Mol) Acetanhydrid wird während 90 Minuten auf Rückflusstemperatur erhitzt und danach auf Natriumbicarbonatlösung gegeben. Das wässrige Gemisch wird zweimal mit je 100 ml Diäthyläther extrahiert, und die vereinigten Extrakte werden dreimal mit konzentrierter Salzsäure ausgeschüttelt. Man neutralisiert die vereinigten wässrigen Phasen mit Natronlauge, extrahiert mit frischem Diäthyläther und wäscht die organische Phase mit Natriumchloridlösung, trocknet sie über wasserfreiem Magnesiumsulfat und dampft sie unter vermindertem Druck ein. Nach Umkristallisieren des Rückstandes aus Diäthyläther/n-Hexan erhält man reines 3-(2-Chlor-6-fluorphenyl)-5-methyl-1-(α,α,α-trifluor -o-tolyl)-1H-1,2,4-triazol, Smp. 102-103,5°C; $^{1}$H-NMR ($CDCl_3$, 60 MHz): 2,40 (s,$C\underline{H}_3$); Massenspektrum: 355(40), 314(52), 159(100).

## Beispiele 58-67

Analog den in Beispiel 56 und 57 beschriebenen Methoden wird das entsprechende Amidrazon der Formel V mit Acetylchlorid bzw. Acetanhydrid umgesetzt, um die in der nachstehenden Tabelle 3 aufgeführten Verbindungen der Formel I herzustellen.

Tabelle 3

| Beispiel | $R^1$ | $R^2$ | $R^3$ | Physikalische Daten |
|---|---|---|---|---|
| 58 | o-Chlorphenyl | 2,6-Difluor-phenyl | Methyl | Smp. 123-124,5°C<br>$^1$H-NMR($CDCl_3$,60 MHz):<br>2,47 (s,$C\underline{H}_3$)<br>Massenspektrum: 305(29), 264(21), 125(100) |
| 59 | o-Tolyl | o-Chlorphenyl | " | Sdp. 160°C/0,015 mmHg (2,00 Pa)<br>$^1$H-NMR($CDCl_3$,60 MHz):<br>2,19 (s,$C\underline{H}_3$), 2,39 (s, $C\underline{H}_3$)<br>Massenspektrum: 283(82), 268(28), 242(9), 105(100) |
| 60 | o-Bromphenyl | " | " | Oel<br>$^1$H-NMR($CDCl_3$,60 MHz):<br>2,42 (s,$C\underline{H}_3$)<br>Massenspektrum: 347/349(61), 306/308(53), 169/171(97) |
| 61 | 2-Aethyl-6-methylphenyl | 2,6-Difluor-phenyl | " | Smp. 96,5-97°C<br>$^1$H-NMR($CDCl_3$,60 MHz):<br>2,05 (s,$C\underline{H}_3$), 2,34 (s,$C\underline{H}_3$), 2,37 (q,$C\underline{H}_2CH_3$)<br>Massenspektrum: 313(67), 298(100) |

Tabelle 3 (Fortsetzung)

| Beispiel | $R^1$ | $R^2$ | $R^3$ | Physikalische Daten |
|---|---|---|---|---|
| 62 | 6-Chlor-o-tolyl | 2,6-Difluor-phenyl | Methyl | Smp. 139°C; $^1$H-NMR ($CDCl_3$,60 MHz): 2,16 (s,C$\underline{H}_3$), 2,40 (s,C$\underline{H}_3$) Massenspektrum: 319(49), 304(40), 139(100) |
| 63 | 2-Chlor-5-tri-fluormethyl-phenyl | " | " | Smp. 81-83°C $^1$H-NMR($CDCl_3$,60 MHz): 2,49 (s,C$\underline{H}_3$) Massenspektrum: 373(18), 332(15), 193(100) |
| 64 | 4-Chlor-2-tri-fluormethyl-phenyl | " | " | Smp. 81-82°C $^1$H-NMR($CDCl_3$,60MHz): 2,40 (s, C$\underline{H}_3$) Massenspektrum: 373(27), 332(23), 193(100) |
| 65 | α,α,α-Trifluor-o-tolyl | 2,6-Dichlor-phenyl | " | Smp. 148-150°C $^1$H-NMR($CDCl_3$,60MHz): 2,41 (s, C$\underline{H}_3$) |
| 66 | 2,4-Dichlor-phenyl | 2-Chlor-6-fluorphenyl | " | Smp. 96°C $^1$H-NMR ($CDCl_3$, 400 MHz): 2,45 (s,C$\underline{H}_3$) Massenspektrum: 355(19), 314(19), 159(100) |
| 67 | o-Chlorphenyl | " | " | Smp. 104°C $^1$H-NMR ($CDCl_3$, 400 MHz): 2,42 (s,C$\underline{H}_3$) Massenspektrum: 321(20), 280(17), 125(100) |

## Beispiel 68

2,5 g (8 mMol) $N^1$-(α,α,α-Trifluor-o-tolyl)-2,6--difluorbenzamidrazon werden in 12,6 g (60 mMol) Trifluoracetanhydrid gelöst, wobei die Temperatur der Lösung auf 35°C ansteigt. Man erhitzt die Lösung 2 Stunden unter stetigem Rühren auf Rückflusstemperatur und giesst sie anschliessend auf Eiswasser. Das wässrige Gemisch wird mit 28%iger Natronlauge alkalisch gestellt und zweimal mit Diäthyläther extrahiert, und die vereinigten organischen Phasen der Reihe nach mit verdünnter Salzsäure, Natriumbicarbonatlösung und Natriumchloridlösung gewaschen, über wasserfreiem Magnesiumsulfat getrocknet und unter vermindertem Druck eingedampft. Nach Umkristallisieren des Rückstandes aus Diäthyläther/n-Hexan erhält man das 3-(2,6-Difluorphenyl)-5-trifluormethyl-1-(α,α,α-trifluor -o-tolyl)-1H-1,2,4-triazol, Smp. 90°C; Massenspektrum: 393(100), 298(26), 159(82).

## Beispiele 69-71

Analog dem in Beispiel 68 beschriebenen Verfahren wird das entsprechende Amidrazon der Formel V mit Trifluoracetanhydrid umgesetzt, um die in der nachstehenden Tabelle 4 aufgeführten Verbindungen der Formel I herzustellen.

<u>Tabelle 4</u>

| Beispiel | $R^1$ | $R^2$ | $R^3$ | Physikalische Daten |
|---|---|---|---|---|
| 69 | o-Chlorphenyl | 2,6-Difluor-phenyl | Trifluor-methyl | Smp. 98-98,5°C Massenspektrum: 359(61), 364(7), 125(100) |
| 70 | 4-Chlor-2-trifluor-methylphenyl | " | " | Sdp. 120°C/0,04 mmHg (5,33 Pa) Massenspektrum: 427(44), 332(8), 193(100) |
| 71 | α,α,α-Trifluor-o-tolyl | 2,6-Dichlor-phenyl | " | Smp. 100°C Massenspektrum: 425(67), 330(20), 159(100) |

## II. Herstellung der Ausgangsmaterialien der Formeln II, III, V und XII:

### Beispiel 72

Das als Ausgangsmaterial zur Herstellung der Verbindung des Beispiels 1 benötigte 1,3-Bis-(o-chlorphenyl)-1,4-dihydro-1H-1,2,4-triazol-5-on kann wie folgt hergestellt werden:

24,0 g (94 mMol) (o-Chlor-α-äthoxybenzyliden)carbaminsäure-äthylester und 16,8 g (94 mMol) o-Chlorphenylhydrazin-hydrochlorid werden in 40 ml 1,2-Dichlorbenzol vorgelegt und mit 13,1 ml (94 mMol) Triäthylamin versetzt, und das Gemisch wird 24 Stunden auf 100°C erhitzt. Während einer weiteren Stunde wird das Gemisch noch auf 162°C erhitzt. Beim Abkühlen wird das Reaktionsgut grösstenteils fest, und man dekantiert das Lösungsmittel ab und schlämmt das Kristallisat in Wasser auf. Das Produkt wird abfiltriert und gut mit Diäthyläther gewaschen. Auf diese Weise erhält man reines 1,3-Bis-(o-chlorphenyl)-1,4-dihydro-1H-1,2,4-triazol-5-on, Smp. 231°C; IR-Spektrum: 1695 $cm^{-1}$.

### Beispiel 73

Das als Ausgangsmaterial zur Herstellung der Verbindung des Beispiels 2 benötigte 3-(o-Chlorphenyl)-1-(α,α,α-trifluor-o-tolyl) -1,4-dihydro-1H-1,2,4-triazol-5-on kann wie folgt hergestellt werden:

Ein Gemisch von 5,0 g (20 mMol) (o-Chlor-α-äthoxybenzyliden)carbaminsäure-äthylester und 3,8 g (21 mMol) o-Trifluormethylphenylhydrazin in 50 ml Tetrachlorkohlenstoff wird während ca. 16 Stunden auf Rückflusstemperatur erhitzt. Anschliessend wird mit 80 ml Methylenchlorid verdünnt, mit 5%iger Natriumbicarbonatlösung gewaschen, über

wasserfreiem Natriumsulfat getrocknet und unter vermindertem Druck eingedampft. Das Rohprodukt wird in Diäthyläther gelöst, und die Lösung durch Filtrieren von unlöslichen Anteilen befreit und mit n-Hexan versetzt. Dabei fallen weisse Kristalle von [o-Chlor-α-(o-trifluormethylphenyl-hydrazino) -benzyliden]carbaminsäure-äthylester aus; Smp. 110-113°C; IR-Spektrum: 1740 $cm^{-1}$.

1,65 g (4,3 mMol) des obigen Zwischenproduktes werden im Kugelrohrofen unter vermindertem Druck während 30 Minuten auf 200°C erhitzt, wobei die anfänglich entstandene Schmelze schliesslich durchkristallisiert. Man erhält auf diese Weise das 3-(o-Chlorphenyl)-1-(α,α,α-trifluor-o-tolyl) -1,4-dihydro-1H-1,2,4-triazol-5-on, Smp. 241-244°C; IR-Spektrum: 1695 $cm^{-1}$.

Das Endprodukt dieses Beispiels kann auch analog dem Verfahren des Beispiels 72, d.h. unter Verwendung des o-Trifluormethylphenylhydrazin-hydrochlorids, hergestellt werden.

## Beispiel 74

Das als Ausgangsmaterial zur Herstellung der Verbindung des Beispiels 3 und 36 benötigte 3-(2,6-Difluorphenyl)-1-(α,α,α-trifluor-o-tolyl) -1,4-dihydro-1H-1,2,4-triazol-5-on kann wie folgt hergestellt werden:

Ein Gemisch von 16,0 g (62,2 mMol) (2,6-Difluor-α-äthoxybenzyliden)carbaminsäure-äthylester und 11,0 g (62,2 mMol) α,α,α-Trifluor-o-tolylhydrazin in 30 ml 1,2-Dichlorbenzol wird unter Abdestillieren des entstehenden Aethanols 18 Stunden auf 135°C erhitzt. Dann wird der ausgefallene Feststoff abfiltriert und mit Diäthyläther gewaschen. Man erhält reines 3-(2,6-Difluorphenyl)-1-(α,α,α-trifluor-o-tolyl) -1,4-dihydro-1H-1,2,4-triazol-5-on, Smp. 290°C; IR-Spektrum (KBr): 1695 $cm^{-1}$;

Massenspektrum: 341(68), 298(26), 159(100).

## Beispiel 75

Das 3-(2,6-Difluorphenyl)-1-(α,α,α-trifluor-o--tolyl) -1,4-dihydro-1H-1,2,4-triazol-5-on kann auch wie folgt hergestellt werden:

2,0 g (6,3 mMol) $N^1$-(α,α,α-Trifluor-o-tolyl)--2,6-difluorbenzamidrazon werden in 8 ml Pyridin vorgelegt und unter gutem Kühlen mit 0,75 g (7,0 mMol) Chlorameisensäure-äthylester versetzt. Man rührt vorerst 1 Stunde nach und erhitzt alsdann während 16 Stunden auf Rückflusstemperatur. Das Reaktionsgemisch wird auf Eiswasser gegossen, und die ausgefallenen Kristalle werden abfiltriert, gut mit Diäthyläther gewaschen und getrocknet. Auf diese Weise erhält man das 3-(2,6-Difluorphenyl)-1-(α,α,α-trifluor-o-tolyl) -1,4-dihydro-1H-1,2,4-triazol-5-on als Kristalle, Smp. 259-260°C; IR-Spektrum (KBr): 1695 $cm^{-1}$.

## Beispiele 76-107

Analog dem in Beispiel 72, 73, 74 oder 75 beschriebenen Verfahren werden die entsprechenden Ausgangsmaterialien der Formeln XII und IV miteinander umgesetzt, um die in der nachstehenden Tabelle 5 aufgeführten Verbindungen der Formel II herzustellen. Auch die diesbezüglichen Endprodukte der Formel I sind in dieser Tabelle angegeben.

Tabelle 5

| Beispiel | Beispiel Nr. des Endproduktes der Formel I | $R^1$ | $R^2$ | Physikalische Daten |
|---|---|---|---|---|
| 76 | 10 | o-Tolyl | o-Chlorphenyl | Smp. 194°C; IR-Spektrum: 1690 $cm^{-1}$; Massenspektrum: 285(95), 148(56), 104(100) |
| 77 | 6 | α,α,α-Trifluor-o-tolyl | 2-Chlor-4-fluorphenyl | Smp. 203°C Massenspektrum: 357(43), 314(19), 159(100) |
| 78 | 4 | o-Chlorphenyl | " | Smp. 208°C Massenspektrum: 323(17), 288(31), 125(100) |
| 79 | 5 | " | 2,6-Difluorphenyl | Smp. 258°C Massenspektrum: 307(20), 272(31), 125(100) |
| 80 | 7 | α,α,α-Trifluor-o-tolyl | 2-Chlor-6-fluorphenyl | Smp. 230°C Massenspektrum: 357(34), 314(20), 159(100) |
| 81 | 8 | o-Chlorphenyl | " | Smp. 223°C IR-Spektrum (KBr): 1658 $cm^{-1}$; Massenspektrum: 323(17), 288(30), 125(100) |

Tabelle 5 (Fortsetzung)

| Beispiel | Beispiel Nr. des Endproduktes der Formel I | $R^1$ | $R^2$ | Physikalische Daten |
|---|---|---|---|---|
| 82 | 9 | 3,4-Dichlorphenyl | 2,6-Difluorphenyl | Smp. über 250°C<br>IR: 1725 $cm^{-1}$<br>Massenspektrum:<br>341(53), 298(7),<br>159(100) |
| 83 | 11 | α,α,α-Trifluor-o-tolyl | o-Jodphenyl | Smp. 213°C |
| 84 | 12 | 2-Chlor-6-methylphenyl | o-Chlorphenyl | Smp. 252°C<br>IR: 1695 $cm^{-1}$<br>Massenspektrum:<br>319(47), 284(100) |
| 85 | 13 | 4-Chlor-o-tolyl | " | Smp. 252-254°C<br>IR: 1695 $cm^{-1}$ |
| 86 | 14 | 3-Chlor-o-tolyl | " | Smp. über 250°C<br>IR: 1695 $cm^{-1}$<br>Massenspektrum:<br>319(75), 138(100) |
| 87 | 15 | 5-Chlor-o-tolyl | " | Smp. über 255°C<br>IR: 1695 $cm^{-1}$<br>Massenspektrum: 319(100) |

Tabelle 5 (Fortsetzung)

| Beispiel | Beispiel Nr. des Endproduktes der Formel I | $R^1$ | $R^2$ | Physikalische Daten |
|---|---|---|---|---|
| 88 | 16 | 2-Chlor-5-trifluormethylphenyl | o-Chlorphenyl | Smp. 198°C<br>IR: 1705 $cm^{-1}$<br>Massenspektrum:<br>373(54), 193(100) |
| 89 | 17 | 2-Chlor-6-fluorphenyl | " | - (nicht isoliert) |
| 90 | 18 | o-Tolyl | 2,6-Difluorphenyl | Smp. 174-176°C<br>IR: 1695/1710 $cm^{-1}$ |
| 91 | 19 | Phenyl | " | Smp. 183°C<br>IR: 1700 $cm^{-1}$ |
| 92 | 20 | 3,5-Dichlorphenyl | " | Smp. über 250°C<br>IR: 1725 $cm^{-1}$<br>Massenspektrum:<br>341(93), 298(23), 159(100) |
| 93 | 21 | 2,4-Dimethylphenyl | " | Smp. 197,5-198,5°C<br>IR: 1700 $cm^{-1}$<br>Massenspektrum: 301(100) |
| 94 | 22 | Phenyl | o-Chlorphenyl | Smp. 182°C |

Tabelle 5 (Fortsetzung)

| Beispiel | Beispiel Nr. des Endprodukes der Formel I | $R^1$ | $R^2$ | Physikalische Daten |
|---|---|---|---|---|
| 95 | 23 | α,α,α-Trifluor-m-tolyl | 2,6-Dimethoxyphenyl | Smp. 187-188°C<br>IR: 1710 $cm^{-1}$<br>$^1H$-NMR [$(CH_3)_2SO$, 60 MHz]: 3,85 (s, 2x$OC\underline{H}_3$) |
| 96 | 24 | " | 2,6-Difluorphenyl | Smp. 219°C<br>IR: 1720 $cm^{-1}$ |
| 97 | 25 | 3-Chlor-o-tolyl | " | Smp. 240°C<br>IR: 1710 $cm^{-1}$<br>Massenspektrum: 321(100) |
| 98 | 26 | " | 2-Chlor-6-fluorphenyl | Smp. 155-160°C |
| 99 | 27 | 2,3-Dimethylphenyl | o-Chlorphenyl | Smp. 201-203°C<br>IR: 1695 $cm^{-1}$<br>Massenspektrum: 299(100), 282(53), 162(64) |
| 100 | 28 | " | 2,6-Difluorphenyl | Smp. 250-254°C<br>IR: 1700 $cm^{-1}$<br>Massenspektrum: 301(100) |
| 101 | 29 | m-Fluorphenyl | o-Chlorphenyl | Smp. 224-226°C<br>IR: 1708 $cm^{-1}$ |
| 102 | 30 | o-Nitrophenyl | " | - (nicht isoliert) |

Tabelle 5 (Fortsetzung)

| Beispiel | Beispiel Nr. des Endproduktes der Formel I | $R^1$ | $R^2$ | Physikalische Daten |
|---|---|---|---|---|
| 103 | 31 | o-Bromphenyl | 2,6-Difluorphenyl | Smp. 280-281°C<br>IR: 1702 $cm^{-1}$ |
| 104 | 32 | o-Methoxyphenyl | o-Chlorphenyl | IR: 1690 $cm^{-1}$ |
| 105 | 33 | o-Fluorphenyl | " | Smp. 185°C<br>IR: 1705 $cm^{-1}$ |
| 106 | 34 | 3-Chlor-2-cyanophenyl | " | Smp. 285°C<br>IR: 1755 $cm^{-1}$<br>Massenspektrum:<br>330(100), 295(63) |
| 107 | 35 | o-Cyanophenyl | " | - (nicht isoliert) |

## Beispiel 108

Der als Ausgangsmaterial zur Herstellung der Verbindungen der Beispiele 38, 45, 46, 54 und 55 benötigte N-Acetyl-o-chlorbenzimidsäure-äthylester kann wie folgt hergestellt werden:

Ein Gemisch von 41 g (22,3 mMol) o-Chlorbenzimidsäure-äthylester und 25 g (25 mMol) Triäthylamin in 500 ml Methylenchlorid wird tropfenweise mit 19,5 g (24,8 mMol) Acetylchlorid versetzt, währenddessen unter Einsatz von Kühlung die Temperatur bei 40°C gehalten wird. Man rührt das Reaktionsgemisch noch 30 Minuten bei Raumtemperatur, dann wäscht man das Gemisch der Reihe nach mit Wasser, 5%iger Natriumbicarbonatlösung und halbgesättigter Kochsalzlösung, trocknet es über wasserfreiem Magnesiumsulfat und dampft es unter vermindertem Druck ein. Nach Destillieren des Rohproduktes bei 110°C/0,16 mmHg (21,33 Pa) erhält man als farblose Flüssigkeit den N-Acetyl-o-chlorbenzimidsäure-äthylester.

## Beispiele 109-118

Analog dem in Beispiel 108 beschriebenen Verfahren wird der entsprechende Benzimidsäure-alkylester der Formel XI mit dem entsprechenden Carbonsäurechlorid umgesetzt, um die in der nachstehenden Tabelle 6 aufgeführten Ausgangsmaterialien der Formel III herzustellen. Auch die diesbezüglichen Endprodukte der Formel I sind in dieser Tabelle angegeben.

**Tabelle 6**

| Beispiel | Beispiel Nr. des Endproduktes der Formel I | $R^2$ | $R^{3'}$ | $R^4$ | Physikalische Daten |
|---|---|---|---|---|---|
| 109 | 39 | 2-Chlor-4-fluorphenyl | Methyl | Aethoxy | Oel $^1H$-NMR($CDCl_3$,60 MHz): 2,07 (s,$CO\underline{CH_3}$), 4,37 (q,$O\underline{CH_2}CH_3$) Massenspektrum: 208(72), 157(85), 43(100) |
| 110 | 40 | o-Fluorphenyl | " | " | Oel $^1H$-NMR($CDCl_3$,60 MHz): 2,14 (d,$J_{F-H}$=1,5Hz, $CO\underline{CH_3}$), 4,31 (q,$O\underline{CH_2}CH_3$) Massenspektrum: 166(26), 123(100) |
| 111 | 41 | 2,4-Dichlorphenyl | " | " | Oel $^1H$-NMR($CDCl_3$,60MHz): 2,05 (s,$CO\underline{CH_3}$), 4,34 (q,$O\underline{CH_2}CH_3$) Massenspektrum: 116(64), 173(66), 43(100) |
| 112 | 42 | o-Bromphenyl | " | " | Oel $^1H$-NMR($CDCl_3$,60 MHz): 2,04 (s,$CO\underline{CH_3}$), 4,34 (q,$O\underline{CH_2}CH_3$) |

<u>Tabelle 6</u> (Fortsetzung)

| Bei-spiel | Beispiel Nr. des Endpro-duktes der Formel I | $R^2$ | $R^{3'}$ | $R^4$ | Physikalische Daten |
|---|---|---|---|---|---|
| 113 | 43 | o-Tolyl | Methyl | Aethoxy | Oel<br>$^1$H-NMR($CDCl_3$,60 MHz): 1,98 (s, $COC\underline{H}_3$), 2,39 (s, $C\underline{H}_3$), 4,31 (q, $OC\underline{H}_2CH_3$) |
| 114 | 44 | o-Methoxy-phenyl | " | " | Oel<br>$^1$H-NMR($CDCl_3$,60MHz): 2,13 (s,$COC\underline{H}_3$), 3,82 (s,$OC\underline{H}_3$), 4,32 (q,$OC\underline{H}_2CH_3$)<br>Massenspektrum: 190(83), 135(100) |
| 115 | 49 | o-Chlorphenyl | Chlor-methyl | " | Oel |
| 116 | 47,50 52,53 | 2,6-Difluor-phenyl | Methyl | " | Sdp. 145°C/20 mmHg (2667 Pa)<br>$^1$H-NMR($CDCl_3$,60MHz): 2,19 (s,$COC\underline{H}_3$), 4,38 (q,$OC\underline{H}_2CH_3$)<br>Massenspektrum: 184(46), 141(100) |

Tabelle 6 (Fortsetzung)

| Beispiel | Beispiel Nr. des Endproduktes der Formel I | $R^2$ | $R^{3'}$ | $R^4$ | Physikalische Daten |
|---|---|---|---|---|---|
| 117 | 48 | α,α,α-Trifluor-o-tolyl | Methyl | Aethoxy | Sdp. 75°C/0,1 mmHg (13,33 Pa) $^1H$-NMR($CDCl_3$,60MHz): 1,96 (s,$COC\underline{H}_3$), 4,32 (q,$OC\underline{H}_2CH_3$) |
| 118 | 51 | o-Jodphenyl | " | " | Oel $^1H$-NMR($CDCl_3$,60MHz): 2,05 (s,$COC\underline{H}_3$), 4,37 (q,$OC\underline{H}_2CH_3$) |

## Beispiel 119

Das als Ausgangsmaterial zur Herstellung der Verbindung des Beispiels 59 benötigte $N^1$-(o-Tolyl)-o-chlorbenzamidrazon kann wie folgt hergestellt werden:

Ein Gemisch von 2,4 g (13 mMol) o-Chlorbenzimidsäure-äthylester, 2,1 g (13 mMol) o-Tolylhydrazin-hydrochlorid und 1,8 ml (13 mMol) Triäthylamin wird in 25 ml Aethylenchlorid während ca. 16 Stunden gerührt. Das Reaktionsgemisch wird dann mit Wasser gewaschen und die organische Phase über wasserfreiem Magnesiumsulfat getrocknet und unter vermindertem Druck eingedampft. Dabei erhält man rohes $N^1$-(o-Tolyl)-o-chlorbenzamidrazon (Smp. 148-150°C nach Kristallisieren aus Methylenchlorid/n-Hexan).

## Beispiel 120

Das als Ausgangsmaterial zur Herstellung der Verbindungen der Beispiele 56 und 68 benötigte $N^1$-(α,α,α-Trifluor-o-tolyl)-2,6-difluorbenzamidrazon kann wie folgt hergestellt werden:

106 g (0,5 Mol) o-Trifluormethylphenylhydrazin-hydrochlorid werden bei 0°C portionenweise in eine Lösung von 93 g (0,5 Mol) 2,6-Difluorbenzimidsäure-äthylester in 250 ml Pyridin eingetragen, und das Reaktionsgemisch wird 4 Stunden bei 0°C und anschliessend weitere 16 Stunden bei Raumtemperatur gerührt. Man verdünnt das Gemisch mit ca. 750 ml Diäthyläther, wäscht es je einmal mit Wasser und gesättigter Natriumchloridlösung, trocknet die organische Phase über wasserfreiem Magnesiumsulfat und dampft sie unter vermindertem Druck ein. Nach Umkristallisieren des Rohproduktes aus Diäthyläther/n-Hexan erhält man das $N^1$-(α,α,α-Trifluor-o-tolyl) -2,6-difluorbenzamidrazon, Smp. 107-108°C.

Beispiele 121-127

Analog den in Beispielen 119 und 120 beschriebenen Methoden wird der entsprechende Benzimidsäure-äthylester der Formel XI' mit dem entsprechenden Phenylhydrazin-hydrochlorid der Formel IV umgesetzt, um die in der nachstehenden Tabelle 7 aufgeführten Ausgangsmaterialien (Amidrazone) der Formel V herzustellen. Auch die diesbezüglichen Endprodukte der Formel I sind in dieser Tabelle angegeben.

Tabelle 7

| Bei-spiel | Beispiel Nr. des Endpro-duktes der Formel I | $R^1$ | $R^2$ | Physikalische Daten |
|---|---|---|---|---|
| 121 | 58,69 | o-Chlorphenyl | 2,6-Difluor-phenyl | Smp. 107-108°C |
| 122 | 60 | o-Bromphenyl | o-Chlorphenyl | Smp. 123-126°C |
| 123 | 61 | 2-Aethyl-6-methylphenyl | 2,6-Difluor-phenyl | - |
| 124 | 62 | 6-Chlor-o-tolyl | " | $^1$H-NMR($CDCl_3$,60MHz): 2,35 (s,C$\underline{H}_3$), 5,10 (breites Signal, N$\underline{H}_2$), 6,15 (breites Signal, N$\underline{H}$) |
| 125 | 63 | 2-Chlor-5-tri-fluormethyl-phenyl | " | $^1$H-NMR($CDCl_3$,60MHz): 4,99 (breites Signal, N$\underline{H}_2$), 6,58 (breites Signal, N$\underline{H}$) |
| 126 | 64,70 | 4-Chlor-2-tri-fluormethyl-phenyl | " | Massenspektrum: 349(34), 332(16), 193(100) |
| 127 | | o-Chlorphenyl | o-Chlorphenyl | Smp. 139-140,5°C |

Beispiel 128

Das als Ausgangsmaterial zur Herstellung der Verbindung des Beispiels 57 benötigte $N^1$-(α,α,α-Trifluor-o--tolyl)-2-chlor -6-fluorbenzamidrazon kann wie folgt hergestellt werden:

Zu einer auf 5°C gekühlten Lösung von 65,4 g (0,37 Mol) o-Trifluormethylphenylhydrazin in 200 ml Pyridin tropft man während 60 Minuten unter gutem Rühren 71,7 g (0,37 Mol) 2-Chlor-6-fluorbenzoylchlorid genügend langsam zu, damit die Temperatur des Reaktionsgemisches 5°C nicht übersteigt. Das Gemisch wird noch 90 Minuten bei Raumtemperatur nachgerührt und anschliessend mit 400 ml Wasser versetzt. Man extrahiert das wässrige Gemisch dreimal mit je 150 ml Diäthyläther und wäscht die vereinigten organischen Phasen der Reihe nach mit 2N Natronlauge und konzentrierter Natriumchloridlösung. Dann wird die organische Phase über wasserfreiem Natriumsulfat getrocknet und unter vermindertem Druck eingedampft, wobei zur Entfernung von überschüssigem Pyridin das Rohprodukt noch zweimal mit wenig Toluol eingedampft wird. Nach Umkristallisieren aus Methylenchlorid/n-Hexan erhält man $N^1$-(α,α,α-Trifluor-o-tolyl)-2-chlor -6-fluorbenzhydrazid, Smp. 147-148°C; Massenspektrum: 332(19), 157(100).

Eine Suspension von 117,4 g (0,35 Mol) des obigen Produktes in 61,4 g (0,40 Mol) Phosphoroxychlorid wird 90 Minuten auf Rückflusstemperatur erhitzt. Danach wird das erkaltete Reaktionsgemisch auf eiskalte Natriumbicarbonatlösung gegossen und das Ganze zweimal mit je 200 ml Diäthyläther extrahiert. Die vereinigten organischen Phasen werden mit Natriumchloridlösung gewaschen, über wasserfreiem Magnesiumsulfat getrocknet und unter vermindertem Druck eingedampft. Nach Aufnahme des Rückstandes in n-Hexan filtriert man die ungelösten Anteile ab und dampft das Filtrat unter vermindertem Druck zur Trockene ein. Auf diese Weise erhält man das $N^1$-(α,α,α-Trifluor-o-tolyl)-2-chlor -6-fluor-

benzhydrazinoylchlorid, Massenspektrum: 350(37), 314(39), 159(100).

89,7 g (0,28 Mol) des obigen Produktes werden in 90 ml Diäthyläther aufgenommen, und die Lösung wird bei -50°C - -40°C unter heftigem Rühren während 10 Minuten mit 90 ml 25%iger wässriger Ammoniaklösung versetzt. Man lässt das Reaktionsgemisch während 90 Minuten auf 0°C kommen und trennt die wässrige Phase ab. Die organische Phase wird dann dreimal mit verdünnter Salzsäure extrahiert, und die vereinigten, wässrigen sauren Extrakte werden mit Natronlauge neutralisiert und anschliessend mit frischem Diäthyläther extrahiert. Man trocknet die organische Phase über wasserfreiem Magnesiumsulfat und dampft sie unter vermindertem Druck ein. Nach Umkristallisieren des Rückstandes aus n-Hexan erhält man reines $N^1$-(α,α,α-Trifluor-o--tolyl)-2-chlor -6-fluorbenzamidrazon, Smp. 132,5°C.

## Beispiele 129-131

Analog der in Beispiel 128 beschriebenen Methode wird das entsprechende Benzoesäurechlorid der Formel XV mit dem entsprechenden Phenylhydrazin der Formel IV umgesetzt, das so erhaltene N-Phenyl-benzhydrazid der Formel XVI mit einem Chlorierungsmittel behandelt, und schliesslich das auf diese Weise erhaltene N-Phenyl-benzhydrazinoylchlorid der Formel XIV einer Aminolyse unterworfen, um die in der nachstehenden Tabelle 8 aufgeführten Ausgangsmaterialien (Amidrazone) der Formel V herzustellen. Auch die diesbezüglichen Endprodukte der Formel I sind in dieser Tabelle angegeben.

Tabelle 8

| Beispiel | Beispiel Nr. des Endproduktes der Formel I | $R^1$ | $R^2$ | Physikalische Daten |
|---|---|---|---|---|
| 129 | 65,71 | α,α,α-Trifluor-o-tolyl | 2,6-Dichlorphenyl | $^1H$-NMR($CDCl_3$, 60 MHz): 4,90 (breites Signal, N$\underline{H}_2$), 6,45 (breites Signal, N$\underline{H}$) Massenspektrum: 347(33), 330(31), 159(100) |
| 130 | 66 | 2,4-Dichlorphenyl | 2-Chlor-6-fluorphenyl | - (nicht isoliert) |
| 131 | 67 | o-Chlorphenyl | " | Smp. 162-163°C |

Auf analoge Weise können auch die Verbindungen der Beispiele 119-127 hergestellt werden.

Beispiel 132

Der als Ausgangsmaterial zur Herstellung der 1,4--Dihydro-1H-1,2,4-triazol-5-one der Beispiele 72, 73, 76, 84-89, 94, 99, 101, 102 und 104-107 benötigte (o-Chlor-α--äthoxybenzyliden)carbaminsäure-äthylester kann wie folgt hergestellt werden:

Ein Gemisch von 40 g (22 mMol) o-Chlorbenzimidsäure--äthylester und 22,2 g (21 mMol) 2,6-Lutidin in 500 ml Petroleumbenzin (Sdp. 100-130°C) wird mit 27,2 g (25 mMol) Chlorameisensäure-äthylester versetzt und das Ganze dann während ca. 16 Stunden auf Rückflusstemperatur erhitzt. Man filtriert anschliessend das ausgefallene Salz ab, dampft das Filtrat unter vermindertem Druck ein und kristallisiert den Rückstand aus Diäthyläther um. Auf diese Weise erhält man den (o-Chlor-α-äthoxybenzyliden)carbaminsäure-äthylester, Smp. 65-66°C; $^{1}$H-NMR ($CDCl_3$), 60 MHz): 4,02 (q,$OC\underline{H}_2CH_3$), 4,58 (q,$OCH_2C\underline{H}_3$); Massenspektrum: 220(100).

Beispiele 133-137

Analog dem in Beispiel 132 beschriebenen Verfahren wird der entsprechende Benzimidsäure-äthylester der Formel XI mit Chlorameisensäure-äthylester umgesetzt, um die in der nachstehenden Tabelle 9 aufgeführten Ausgangsmaterialien der Formel XII herzustellen. Auch die diesbezüglichen Endprodukte der Formel II sind in dieser Tabelle angegeben.

Tabelle 9

| Beispiel | Beispiel Nr. des Endproduktes der Formel II | $R^2$ | $R^4$ | $R^6$ | Physikalische Daten |
|---|---|---|---|---|---|
| 133 | 74,79,82, 90-93,96, 97,100,103 | 2,6-Difluorphenyl | Aethoxy | Aethoxy | Sdp. 115°C/0,8 mmHg (106,67 Pa) $^1$H-NMR($CDCl_3$,60 MHz): 4,14 (q,$OC\underline{H}_2CH_3$), 4,44 (q,$OC\underline{H}_2CH_3$) |
| 134 | 77,78 | 2-Chlor-4-fluorphenyl | " | " | Oel |
| 135 | 80,81,98 | 2-Chlor-6-fluorphenyl | " | " | Sdp. 100°C/0,02 mmHg (2,67 Pa) $^1$H-NMR($CDCl_3$, 60 MHz): 4,07 (q,$OC\underline{H}_2CH_3$), 4,45 (q, $OC\underline{H}_2CH_3$) |
| 136 | 83 | o-Jodphenyl | " | " | Oel $^1$H-NMR($CDCl_3$, 60 MHz): 3,99 (q, $OC\underline{H}_2CH_3$), 4,39 (q, $OC\underline{H}_2CH_3$) |
| 137 | 95 | 2,6-Dimethoxyphenyl | " | " | Smp. 73-74°C $^1$H-NMR($CDCl_3$, 60 MHz): 3,81 (s, $OC\underline{H}_3$), 4,01 (q, $OC\underline{H}_2CH_3$), 4,40 (q, $OC\underline{H}_2CH_3$) |

III. Formulierungsbeispiele:

## Beispiel 138

Ein emulgierbares Konzentrat hat folgende Zusammensetzung:

| | | g/Liter |
|---|---|---|
| Verbindung der Formel I (Wirkstoff) | | 250 |
| Polyarylphenol-(18)äthoxylat | (Emulgatoren) | 300 |
| Polyvinylpyrrolidon | (Emulgatoren) | 20 |
| Isotridecylalkohol | (Emulgatoren) | 20 |
| N-Methyl-2-pyrrolidon (Lösungsmittel) | | ad 1 Liter |

Der Wirkstoff und die Emulgatoren werden im Lösungsmittel gelöst. Nach Verdünnen mit Wasser ergibt das so entstandene emulgierbare Konzentrat eine Emulsion, die sich gut als Spritzbrühe eignet.

## Beispiel 139

Ein emulgierbares Konzentrat hat folgende Zusammensetzung:

| | | g/Liter |
|---|---|---|
| Verbindung der Formel I (Wirkstoff) | | 250 |
| Nonylphenol-(10) äthoxylat | (Emulgatoren) | 75 |
| Calcium-Dodecylbenzolsulfonat | (Emulgatoren) | 25 |
| N-Methyl-2-pyrrolidon | (Lösungsmittel) | 400 |
| Gemisch aus Mono-, Di- und Tri(nieder alkyl)benzolen | (Lösungsmittel) | ad 1000 ml |

Der Wirkstoff und die beiden Emulgatoren werden im ersten Lösungsmittel gelöst, und anschliessend wird das Volumen durch Zusatz des zweiten Lösungsmittels auf 1 l gebracht. Nach Verdünnen mit Wasser ergibt das so ent-

standende emulgierbare Konzentrat eine Emulsion, die sich gut als Spritzbrühe eignet.

## Beispiel 140

Ein Spritzpulver hat folgende Zusammensetzung:

| | | Gewichtsprozent |
|---|---|---|
| Verbindung der Formel I (Wirkstoff) | | 50 |
| Natrium-laurylsulfat (Netz-/Dispergiermittel) | | 1 |
| Natrium-lignosulfonat (Dispergiermittel) | | 2 |
| Hydratisierte Kieselsäure (ca. 87% $SiO_2$) | (inerte, pulverförmige Trägerstoffe) | 5 |
| Kaolin (hauptsächlich $Al_2(Si_2O_5)(OH)_4$) | | 42 |
| | | 100 |

Der Wirkstoff wird mit den übrigen Formulierungskomponenten in einer geeigneten Vorrichtung homogen vermischt. Das entstandene Pulver wird nun in einem geeigneten Mahlaggregat (z.B. Stiften-, Hammer-, Kugel- oder Luftstrahlmühle) auf eine für eine optimale biologische Wirksamkeit notwendige Teilchengrösse feingemahlen und hernach nochmals gemischt. Das nun vorliegende Spritzpulver wird durch Wasser spontan benetzt und ergibt gut schwebefähige, gebrauchsfertige Spritzbrühen.

## Patentansprüche

1. Verbindungen der allgemeinen Formel

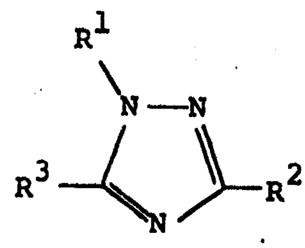

I

worin

$R^1$ gegebenenfalls mit 1 bis 3 Chloratomen, einem Bromatom, einem Jodatom, 1 bis 3 Fluoratomen, 1 oder 2 $C_{1-2}$--Alkylgruppen, 1 oder 2 Halogenmethylgruppen, einer $C_{1-2}$-Alkoxygruppe, einer $C_{1-2}$-Halogenalkoxygruppe, einer Nitrogruppe und/oder einer Cyanogruppe substituiertes Phenyl,

$R^2$ mit 1 oder 2 Chloratomen, einem Bromatom, einem Jodatom, 1 bis 3 Fluoratomen, 1 oder 2 $C_{1-2}$-Alkylgruppen, einer Halogenmethylgruppe und/oder 1 oder 2 Methoxygruppen substituiertes Phenyl, wobei sich mindestens einer der Substituenten in o-Stellung befindet, und

$R^3$ Halogen, Methyl oder Halogenmethyl bedeuten,

sowie die Säureadditionssalze der Verbindungen der Formel I.

2. Verbindungen nach Anspruch 1, worin $R^1$ substituiertes Phenyl bedeutet, wobei sich mindestens einer der Substituenten in o-Stellung befindet.

3. Verbindungen nach Anspruch 1 oder 2, worin $R^1$ eine mono- oder disubstituierte Phenylgruppe bedeutet, in der die Substituenten ein oder zwei Fluoratome, ein oder zwei Chloratome, ein Bromatom, ein Jodatom, eine oder zwei $C_{1-2}$--Alkylgruppen und/oder eine Trifluormethylgruppe sind.

4. Verbindungen nach Anspruch 2 oder 3, worin der Substituent in o-Stellung Fluor, Chlor, Brom, Methyl oder Trifluormethyl ist.

5. Verbindungen nach einem der Ansprüche 1 bis 4, worin $R^2$ eine mono- oder disubstituierte Phenylgruppe bedeutet, in der die Substituenten ein oder zwei Fluoratome, ein oder zwei Chloratome, ein Bromatom und/oder ein Jodatom sind.

6. Verbindungen nach Anspruch 5, worin $R^2$ o-Chlorphenyl, 2-Chlor-4-fluorphenyl, 2-Chlor-6-fluorphenyl oder 2,6-Difluorphenyl bedeutet.

7. Verbindungen nach einem der Ansprüche 1 bis 6, worin $R^3$ Fluor, Chlor, Brom oder Methyl bedeutet.

8. Verbindungen nach Anspruch 7, worin $R^3$ Chlor bedeutet.

9. Eine Verbindung nach Anspruch 2, ausgewählt aus:

1,3-Bis-(o-chlorphenyl)-5-chlor-1H-1,2,4-triazol,
5-Chlor-3-(o-chlorphenyl)-1-(α,α,α-trifluor-o-tolyl)-1H-1,2,4-triazol,
5-Chlor-3-(2,6-difluorphenyl)-1-(α,α,α-trifluor-o-tolyl)-1H-1,2,4-triazol,
5-Chlor-1-(o-chlorphenyl)-3-(2-chlor-4-fluorphenyl)-1H-1,2,4-triazol,
5-Chlor-1-(o-chlorphenyl)-3-(2,6-difluorphenyl)-1H-1,2,4-triazol,
5-Chlor-3-(2-chlor-4-fluorphenyl)-1-(α,α,α-trifluor-o-tolyl)-1H-1,2,4-triazol,
5-Chlor-3-(2-chlor-6-fluorphenyl)-1-(α,α,α-trifluor-o-tolyl)-1H-1,2,4-triazol,
5-Chlor-1-(o-chlorphenyl)-3-(2-chlor-6-fluorphenyl)-1H-1,2,4-triazol,

5-Brom-3-(2,6-difluorphenyl)-1-(α,α,α-trifluor-o-tolyl)-1H-1,2,4-triazol,

3-(o-Chlorphenyl)-5-methyl-1-(α,α,α-trifluor-o-tolyl)-1H-1,2,4-triazol,

3-(2-Chlor-4-fluorphenyl)-5-methyl-1-(α,α,α-trifluor-o-tolyl)-1H-1,2,4-triazol,

3-(2,6-Difluorphenyl)-5-methyl-1-(α,α,α-trifluor-o-tolyl)-1H-1,2,4-triazol und

3-(2-Chlor-6-fluorphenyl)-5-methyl-1-(α,α,α-trifluor-o-tolyl)-1H-1,2,4-triazol.

10. Eine Verbindung nach Anspruch 1, ausgewählt aus:

5-Chlor-3-(o-chlorphenyl)-1-(o-tolyl)-1H-1,2,4-triazol,

5-Chlor-3-(o-chlorphenyl)-1-(3-chlor-o-tolyl)-1H-1,2,4-triazol,

5-Chlor-3-(o-chlorphenyl)-1-(5-chlor-o-tolyl)-1H-1,2,4-triazol,

5-Chlor-3-(2,6-difluorphenyl)-1-(o-tolyl)-1H-1,2,4-triazol,

5-Chlor-3-(2,6-difluorphenyl)-1-phenyl-1H-1,2,4-triazol,

5-Chlor-3-(2,6-difluorphenyl)-1-(3-chlor-o-tolyl)-1H-1,2,4-triazol,

5-Chlor-3-(2-chlor-6-fluorphenyl)-1-(3-chlor-o-tolyl)-1H-1,2,4-triazol,

1-(o-Bromphenyl)-5-chlor-3-(2,6-difluorphenyl)-1H-1,2,4-triazol,

5-Chlor-3-(o-chlorphenyl)-1-(o-fluorphenyl)-1H-1,2,4-triazol und

3-(2-Chlor-6-fluorphenyl)-5-fluor-1-(α,α,α-trifluor-o-tolyl)-1H-1,2,4-triazol.

11. Schädlingsbekämpfungsmittel, dadurch gekennzeichnet, dass es eine wirksame Menge mindestens einer Verbindung der allgemeinen Formel

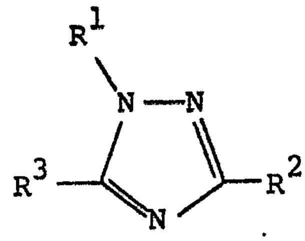

I

worin

$R^1$ gegebenenfalls mit 1 bis 3 Chloratomen, einem Bromatom, einem Jodatom, 1 bis 3 Fluoratomen, 1 oder 2 $C_{1-2}$-Alkylgruppen, 1 oder 2 Halogenmethylgruppen, einer $C_{1-2}$-Alkoxygruppe, einer $C_{1-2}$-Halogenalkoxygruppe, einer Nitrogruppe und/oder einer Cyanogruppe substituiertes Phenyl,

$R^2$ mit 1 oder 2 Chloratomen, einem Bromatom, einem Jodatom, 1 bis 3 Fluoratomen, 1 oder 2 $C_{1-2}$-Alkylgruppen, einer Halogenmethylgruppe und/oder 1 oder 2 Methoxygruppen substituiertes Phenyl, wobei sich mindestens einer der Substituenten in o-Stellung befindet, und

$R^3$ Halogen, Methyl oder Halogenmethyl bedeuten,

oder eines Säureadditionssalzes davon, sowie Formulierungshilfsstoffe enthält.

12. Schädlingsbekämpfungsmittel nach Anspruch 11, worin $R^1$ substituiertes Phenyl bedeutet, wobei sich mindestens einer der Substituenten in o-Stellung befindet.

13. Schädlingsbekämpfungsmittel nach Anspruch 12, dadurch gekennzeichnet, dass es eine wirksame Menge mindestens einer aus der Gruppe

1,3-Bis-(o-chlorphenyl)-5-chlor-1H-1,2,4-triazol,

5-Chlor-3-(o-chlorphenyl)-1-(α,α,α-trifluor-o-tolyl)-1H-1,2,4-triazol,

5-Chlor-3-(2,6-difluorphenyl)-1-(α,α,α-trifluor-o-tolyl)-1H-1,2,4-triazol,

5-Chlor-1-(o-chlorphenyl)-3-(2-chlor-4-fluorphenyl)-1H-1,2,4-triazol,

5-Chlor-1-(o-chlorphenyl)-3-(2,6-difluorphenyl)-1H-1,2,4-triazol,

5-Chlor-3-(2-chlor-4-fluorphenyl)-1-(α,α,α-trifluor-o-tolyl)-1H-1,2,4-triazol,

5-Chlor-3-(2-chlor-6-fluorphenyl)-1-(α,α,α-trifluor-o-tolyl)-1H-1,2,4-triazol,

5-Chlor-1-(o-chlorphenyl)-3-(2-chlor-6-fluorphenyl)-1H-1,2,4-triazol,

5-Brom-3-(2,6-difluorphenyl)-1-(α,α,α-trifluor-o-tolyl)-1H-1,2,4-triazol,

3-(o-Chlorphenyl)-5-methyl-1-(α,α,α-trifluor-o-tolyl)-1H-1,2,4-triazol,

3-(2-Chlor-4-fluorphenyl)-5-methyl-1-(α,α,α-trifluor-o-tolyl)-1H-1,2,4-triazol,

3-(2,6-Difluorphenyl)-5-methyl-1-(α,α,α-trifluor-o-tolyl)-1H-1,2,4-triazol und

3-(2-Chlor-6-fluorphenyl)-5-methyl-1-(α,α,α-trifluor-o-tolyl)-1H-1,2,4-triazol

ausgewählten Verbindung sowie Formulierungshilfsstoffe enthält.

14. Schädlingsbekämpfungsmittel nach Anspruch 11, dadurch gekennzeichnet, dass es eine wirksame Menge mindestens einer aus der Gruppe

5-Chlor-3-(o-chlorphenyl)-1-(o-tolyl)-1H-1,2,4-triazol,

5-Chlor-3-(o-chlorphenyl)-1-(3-chlor-o-tolyl)-1H-1,2,4-triazol,

5-Chlor-3-(o-chlorphenyl)-1-(5-chlor-o-tolyl)-1H-1,2,4-triazol,

5-Chlor-3-(2,6-difluorphenyl)-1-(o-tolyl)-1H-1,2,4-triazol,

5-Chlor-3-(2,6-difluorphenyl)-1-phenyl-1H-1,2,4-triazol,

5-Chlor-3-(2,6-difluorphenyl)-1-(3-chlor-o-tolyl)-1H-1,2,4-triazol,

5-Chlor-3-(2-chlor-6-fluorphenyl)-1-(3-chlor-o-tolyl)-1H-1,2,4-triazol,

1-(o-Bromphenyl)-5-chlor-3-(2,6-difluorphenyl)-1H-1,2,4-triazol,

5-Chlor-3-(o-chlorphenyl)-1-(o-fluorphenyl)-1H-1,2,4-triazol und

3-(2-Chlor-6-fluorphenyl)-5-fluor-1-(α,α,α-trifluor-o-tolyl)-1H-1,2,4-triazol

ausgewählten Verbindung sowie Formulierungshilfsstoffe enthält.

15. Verfahren zur Herstellung von Verbindungen der allgemeinen Formel

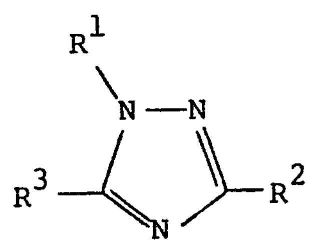

I

worin

$R^1$ gegebenenfalls mit 1 bis 3 Chloratomen, einem Bromatom, einem Jodatom, 1 bis 3 Fluoratomen, 1 oder 2 $C_{1-2}$-Alkylgruppen, 1 oder 2 Halogenmethylgruppen, einer $C_{1-2}$-Alkoxygruppe, einer $C_{1-2}$-Halogenalkoxygruppe, einer Nitrogruppe und/oder einer Cyanogruppe substituiertes Phenyl,

$R^2$ mit 1 oder 2 Chloratomen, einem Bromatom, einem Jodatom, 1 bis 3 Fluoratomen, 1 oder 2 $C_{1-2}$-Alkylgruppen, einer Halogenmethylgruppe und/oder 1 oder 2 Methoxygruppen substituiertes Phenyl, wobei sich mindestens einer der Substituenten in o-Stellung befindet, und

$R^3$ Halogen, Methyl oder Halogenmethyl bedeuten,

und von ihren Säureadditionssalzen, dadurch gekennzeichnet,

dass man

a) zwecks Herstellung derjenigen Verbindungen der Formel I, in denen $R^3$ Chlor oder Brom bedeutet, ein 1,4-Dihydro--1H-1,2,4-triazol-5-on der allgemeinen Formel

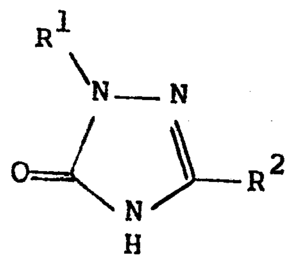

II

worin
$R^1$ und $R^2$ die oben angegebenen Bedeutungen besitzen, mit einem Chlorierungs- oder Bromierungsmittel behandelt,

b) zwecks Herstellung derjenigen Verbindungen der Formel I, in denen $R^3$ Fluor oder Jod bedeutet, ein 5-Chlor-1,2,4--triazol der allgemeinen Formel

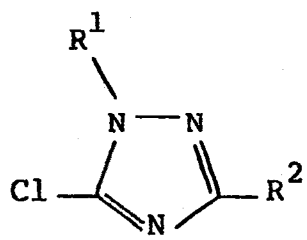

I'

worin $R^1$ und $R^2$ die oben angegebenen Bedeutungen besitzen,

einer Halogenaustauschreaktion unterwirft,

c) zwecks Herstellung derjenigen Verbindungen der Formel I, in denen $R^3$ Methyl oder Halogenmethyl bedeutet, einen N-Acyl-benzimidsäure(thiol)ester bzw. ein N-Acyl-benzimidsäureamid der allgemeinen Formel

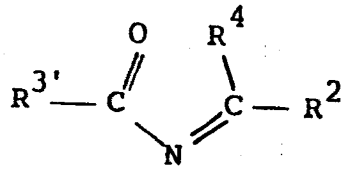

III

worin

$R^{3'}$ Methyl oder Halogenmethyl

und

$R^4$ nieder Alkoxy, nieder Alkylthio oder Di(nieder alkyl)-amino bedeuten

und

$R^2$ die oben angegebene Bedeutung besitzt,

mit einem Phenylhydrazin der allgemeinen Formel

$$R^1\text{-NH-NH}_2 \qquad \text{IV}$$

worin $R^1$ die oben angegebene Bedeutung besitzt,

oder mit einem Säureadditionssalz davon umsetzt, oder

d) zwecks Herstellung derjenigen Verbindungen der Formel I, in denen $R^3$ Methyl oder Halogenmethyl bedeutet, ein Amidrazon der allgemeinen Formel

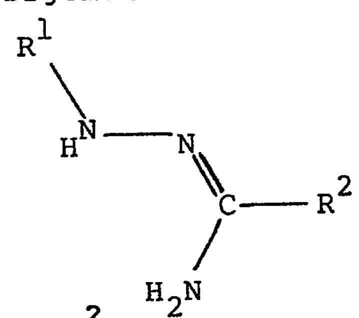

V

worin $R^1$ und $R^2$ die oben angegebenen Bedeutungen besitzen,

oder ein Säureadditionssalz davon mit einer Carbonsäure der allgemeinen Formel

$$R^{3'}\text{-COOH} \qquad \text{VI}$$

worin $R^{3'}$ die oben angegebene Bedeutung besitzt,

oder mit einem reaktionsfähigen Derivat davon umsetzt,

und erwünschtenfalls eine so erhaltene Verbindung der Formel I durch Umsetzung mit einer Säure in das entsprechende Säureadditionssalz überführt.

16. Verfahren nach Anspruch 15 zur Herstellung derjenigen Verbindungen der Formel I, in denen $R^1$ substituiertes Phenyl bedeutet, wobei sich mindestens einer der Substituenten in o-Stellung befindet.

17. Verfahren zur Bekämpfung von Schädlingen, dadurch gekennzeichnet, dass man das zu schützende Gut oder die Schädlinge selbst mit einer wirksamen Menge einer Verbindung gemäss Anspruch 1 oder 10 bzw. eines Mittels gemäss Anspruch 11 oder 14 behandelt.

18. Verfahren zur Bekämpfung von Schädlingen, dadurch gekennzeichnet, dass man das zu schützende Gut oder die Schädlinge selbst mit einer wirksamen Menge einer Verbindung gemäss einem der Ansprüche 2 bis 9 bzw. eines Mittels gemäss Anspruch 12 oder 13 behandelt.

19. Verwendung einer Verbindung gemäss Anspruch 1 oder 10 bzw. eines Mittels gemäss Anspruch 11 oder 14 zur Bekämpfung von Schädlingen.

20. Verwendung einer Verbindung gemäss einem der Ansprüche 2 bis 9 bzw. eines Mittels gemäss Anspruch 12 oder 13 zur Bekämpfung von Schädlingen.

***

## Patentansprüche für AT

1. Schädlingsbekämpfungsmittel, dadurch gekennzeichnet, dass es eine wirksame Menge mindestens einer Verbindung der allgemeinen Formel

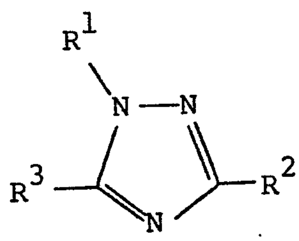

I

worin

$R^1$ gegebenenfalls mit 1 bis 3 Chloratomen, einem Bromatom, einem Jodatom, 1 bis 3 Fluoratomen, 1 oder 2 $C_{1-2}$--Alkylgruppen, 1 oder 2 Halogenmethylgruppen, einer $C_{1-2}$-Alkoxygruppe, einer $C_{1-2}$-Halogenalkoxygruppe, einer Nitrogruppe und/oder einer Cyanogruppe substituiertes Phenyl,

$R^2$ mit 1 oder 2 Chloratomen, einem Bromatom, einem Jodatom, 1 bis 3 Fluoratomen, 1 oder 2 $C_{1-2}$-Alkylgruppen, einer Halogenmethylgruppe und/oder 1 oder 2 Methoxygruppen substituiertes Phenyl, wobei sich mindestens einer der Substituenten in o-Stellung befindet, und

$R^3$ Halogen, Methyl oder Halogenmethyl bedeuten,

oder eines Säureadditionssalzes davon, sowie Formulierungshilfsstoffe enthält.

2. Schädlingsbekämpfungsmittel nach Anspruch 1, worin $R^1$ substituiertes Phenyl bedeutet, wobei sich mindestens einer der Substituenten in o-Stellung befindet.

3. Schädlingsbekämpfungsmittel nach Anspruch 1 oder 2, worin $R^1$ eine mono- oder disubstituierte Phenylgruppe bedeutet, in der die Substituenten ein oder zwei Fluoratome, ein oder zwei Chloratome, ein Bromatom, ein Jodatom, eine

oder zwei $C_{1-2}$-Alkylgruppen und/oder eine Trifluormethylgruppe sind.

4. Schädlingsbekämpfungsmittel nach Anspruch 2 oder 3, worin der Substituent in o-Stellung Fluor, Chlor, Brom, Methyl oder Trifluormethyl ist.

5. Schädlingsbekämpfungsmittel nach einem der Ansprüche 1 bis 4, worin $R^2$ eine mono- oder disubstituierte Phenylgruppe bedeutet, in der die Substituenten ein oder zwei Fluoratome, ein oder zwei Chloratome, ein Bromatom und/oder ein Jodatom sind.

6. Schädlingsbekämpfungsmittel nach Anspruch 5, worin $R^2$ o-Chlorphenyl, 2-Chlor-4-fluorphenyl, 2-Chlor-6-fluorphenyl oder 2,6-Difluorphenyl bedeutet.

7. Schädlingsbekämpfungsmittel nach einem der Ansprüche 1 bis 6, worin $R^3$ Fluor, Chlor, Brom oder Methyl bedeutet.

8. Schädlingsbekämpfungsmittel nach Anspruch 7, worin $R^3$ Chlor bedeutet.

9. Schädlingsbekämpfungsmittel nach Anspruch 2, dadurch gekennzeichnet, dass es eine wirksame Menge mindestens einer aus der Gruppe

1,3-Bis-(o-chlorphenyl)-5-chlor-1H-1,2,4-triazol,

5-Chlor-3-(o-chlorphenyl)-1-(α,α,α-trifluor-o-tolyl)-1H-1,2,4-triazol,

5-Chlor-3-(2,6-difluorphenyl)-1-(α,α,α-trifluor-o-tolyl)-1H-1,2,4-triazol,

5-Chlor-1-(o-chlorphenyl)-3-(2-chlor-4-fluorphenyl)-1H-1,2,4-triazol,

5-Chlor-1-(o-chlorphenyl)-3-(2,6-difluorphenyl)-1H-1,2,4-triazol,

5-Chlor-3-(2-chlor-4-fluorphenyl)-1-(α,α,α-trifluor-o-tolyl)-1H-1,2,4-triazol,

5-Chlor-3-(2-chlor-6-fluorphenyl)-1-(α,α,α-trifluor-o-tolyl)-1H-1,2,4-triazol,

5-Chlor-1-(o-chlorphenyl)-3-(2-chlor-6-fluorphenyl)-1H-1,2,4-triazol,

5-Brom-3-(2,6-difluorphenyl)-1-(α,α,α-trifluor-o-tolyl)-1H-1,2,4-triazol,

3-(o-Chlorphenyl)-5-methyl-1-(α,α,α-trifluor-o-tolyl)-1H-1,2,4-triazol,

3-(2-Chlor-4-fluorphenyl)-5-methyl-1-(α,α,α-trifluor-o-tolyl)-1H-1,2,4-triazol,

3-(2,6-Difluorphenyl)-5-methyl-1-(α,α,α-trifluor-o-tolyl)-1H-1,2,4-triazol und

3-(2-Chlor-6-fluorphenyl)-5-methyl-1-(α,α,α-trifluor-o-tolyl)-1H-1,2,4-triazol

ausgewählten Verbindung sowie Formulierungshilfsstoffe enthält.

10. Schädlingsbekämpfungsmittel nach Anspruch 1, dadurch gekennzeichnet, dass es eine wirksame Menge mindestens einer aus der Gruppe

5-Chlor-3-(o-chlorphenyl)-1-(o-tolyl)-1H-1,2,4-triazol,

5-Chlor-3-(o-chlorphenyl)-1-(3-chlor-o-tolyl)-1H-1,2,4-triazol,

5-Chlor-3-(o-chlorphenyl)-1-(5-chlor-o-tolyl)-1H-1,2,4-triazol,

5-Chlor-3-(2,6-difluorphenyl)-1-(o-tolyl)-1H-1,2,4-triazol,

5-Chlor-3-(2,6-difluorphenyl)-1-phenyl-1H-1,2,4-triazol,

5-Chlor-3-(2,6-difluorphenyl)-1-(3-chlor-o-tolyl)-1H-1,2,4-triazol,

5-Chlor-3-(2-chlor-6-fluorphenyl)-1-(3-chlor-o-tolyl)-1H-1,2,4-triazol,

1-(o-Bromphenyl)-5-chlor-3-(2,6-difluorphenyl)-1H-1,2,4-triazol,

5-Chlor-3-(o-chlorphenyl)-1-(o-fluorphenyl)-1H-1,2,4-triazol und

3-(2-Chlor-6-fluorphenyl)-5-fluor-1-(α,α,α-trifluor-o-tolyl)-1H-1,2,4-triazol

ausgewählten Verbindung sowie Formulierungshilfsstoffe enthält.

11. Verfahren zur Herstellung von Verbindungen der allgemeinen Formel

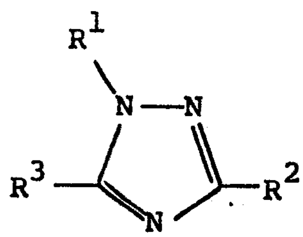

I

worin

$R^1$ gegebenenfalls mit 1 bis 3 Chloratomen, einem Bromatom, einem Jodatom, 1 bis 3 Fluoratomen, 1 oder 2 $C_{1-2}$--Alkylgruppen, 1 oder 2 Halogenmethylgruppen, einer $C_{1-2}$-Alkoxygruppe, einer $C_{1-2}$-Halogenalkoxygruppe, einer Nitrogruppe und/oder einer Cyanogruppe substituiertes Phenyl,

$R^2$ mit 1 oder 2 Chloratomen, einem Bromatom, einem Jodatom, 1 bis 3 Fluoratomen, 1 oder 2 $C_{1-2}$-Alkylgruppen, einer Halogenmethylgruppe und/oder 1 oder 2 Methoxygruppen substituiertes Phenyl, wobei sich mindestens einer der Substituenten in o-Stellung befindet, und

$R^3$ Halogen, Methyl oder Halogenmethyl bedeuten,

und von ihren Säureadditionssalzen, dadurch gekennzeichnet, dass man

a) zwecks Herstellung derjenigen Verbindungen der Formel I, in denen $R^3$ Chlor oder Brom bedeutet, ein 1,4-Dihydro--1H-1,2,4-triazol-5-on der allgemeinen Formel

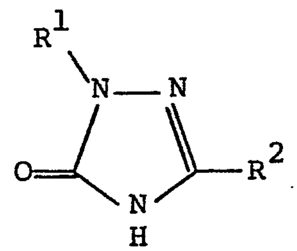

II

worin
$R^1$ und $R^2$ die oben angegebenen Bedeutungen besitzen,
mit einem Chlorierungs- oder Bromierungsmittel behandelt,

b) zwecks Herstellung derjenigen Verbindungen der Formel I, in denen $R^3$ Fluor oder Jod bedeutet, ein 5-Chlor-1,2,4--triazol der allgemeinen Formel

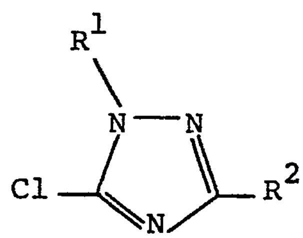

I'

worin $R^1$ und $R^2$ die oben angegebenen Bedeutungen besitzen,

einer Halogenaustauschreaktion unterwirft,

c) zwecks Herstellung derjenigen Verbindungen der Formel I, in denen $R^3$ Methyl oder Halogenmethyl bedeutet, einen N-Acyl-benzimidsäure(thiol)ester bzw. ein N-Acyl-benzimidsäureamid der allgemeinen Formel

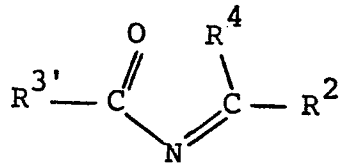

III

worin
$R^{3'}$ Methyl oder Halogenmethyl
und
$R^4$ nieder Alkoxy, nieder Alkylthio oder Di(nieder alkyl)-amino bedeuten

und

$R^2$ die oben angegebene Bedeutung besitzt, mit einem Phenylhydrazin der allgemeinen Formel

$$R^1\text{-NH-NH}_2 \qquad IV$$

worin $R^1$ die oben angegebene Bedeutung besitzt, oder mit einem Säureadditionssalz davon umsetzt, oder

d) zwecks Herstellung derjenigen Verbindungen der Formel I, in denen $R^3$ Methyl oder Halogenmethyl bedeutet, ein Amidrazon der allgemeinen Formel

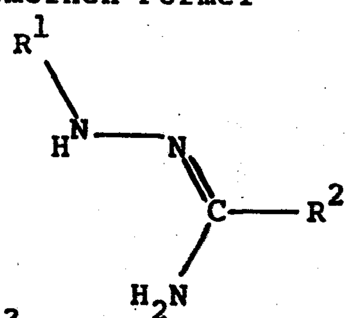

V

worin $R^1$ und $R^2$ die oben angegebenen Bedeutungen besitzen,

oder ein Säureadditionssalz davon mit einer Carbonsäure der allgemeinen Formel

$$R^{3'}\text{-COOH} \qquad VI$$

worin $R^{3'}$ die oben angegebene Bedeutung besitzt, oder mit einem reaktionsfähigen Derivat davon umsetzt,

und erwünschtenfalls eine so erhaltene Verbindung der Formel I durch Umsetzung mit einer Säure in das entsprechende Säureadditionssalz überführt.

12. Verfahren nach Anspruch 11 zur Herstellung derjenigen Verbindungen der Formel I, in denen $R^1$ substituiertes Phenyl bedeutet, wobei sich mindestens einer der Substituenten in o-Stellung befindet.

13. Verfahren zur Herstellung eines Schädlingsbekämpfungsmittels, dadurch gekennzeichnet, dass man mindestens eine der in Anspruch 1 genannten Verbindungen mit Formulierungshilfsstoffen vermischt.

14. Verfahren zur Herstellung eines Schädlingsbekämpfungsmittels, dadurch gekennzeichnet, dass man mindestens eine der in Anspruch 2 genannten Verbindungen mit Formulierungshilfsstoffen vermischt.

15. Verfahren zur Bekämpfung von Schädlingen, dadurch gekennzeichnet, dass man das zu schützende Gut oder die Schädlinge selbst mit einer wirksamen Menge mindestens einer in Anspruch 1 oder 10 genannten Verbindung bzw. eines Mittels gemäss Anspruch 1 oder 10 behandelt.

16. Verfahren zur Bekämpfung von Schädlingen, dadurch gekennzeichnet, dass man das zu schützende Gut oder die Schädlinge selbst mit einer wirksamen Menge mindestens einer in einem der Ansprüche 2 bis 9 genannten Verbindung bzw. eines Mittels gemäss einem dieser Ansprüche behandelt.

17. Verwendung einer in Anspruch 1 oder 10 genannten Verbindung bzw. eines Mittels gemäss Anspruch 1 oder 10 zur Bekämpfung von Schädlingen.

18. Verwendung einer in einem der Ansprüche 2 bis 9 genannten bzw. eines Mittels gemäss einem dieser Ansprüche zur Bekämpfung von Schädlingen.

***